# EUROPEAN PATENT SPECIFICATION

(11) **EP 2 398 500 B1**
(45) Date of publication and mention of the grant of the patent: **13.03.2019**
(21) Application number: 10705204.5
(22) Date of filing: 19.02.2010
(51) Int. Cl.: A61K 47/69, A61K 47/54, A61P 35/00, A61P 25/28

(54) **GLUTATHIONE-BASED DRUG DELIVERY SYSTEM**
ARZNEIABGABESYSTEM AUF GLUTATHION-BASIS
SUSTÈME D'ADMINISTRATION DE MÉDICAMENT À BASE DE GLUTATHIONNE

(30) Priority: 20.02.2009 US 154083 P
(43) Date of publication of application: 28.12.2011
(60) Divisional application: 18177039.7
(73) Proprietor: 2-BBB Medicines B.V., 2333 CH Leiden (NL)
(72) Inventor: GAILLARD, Pieter Jaap, NL-2311 KE Leiden (NL)
(74) Representative: Nederlandsch Octrooibureau
(86) International application number: PCT/NL2010/050082
(87) International publication number: WO 2010/095940

(56) References cited:
- WO-A2-00/75668
- WO-A2-2004/109279
- WO-A2-2007/076323
- WO-A2-2007/149686
- WO-A2-2008/084402
- WO-A2-2008/118013
- JP-A- 63 002 922
- US-A- 5 773 236
- US-A1- 2002 098 999
- US-A1- 2002 192 719
- US-A1- 2005 130 904
- US-A1- 2007 004 639
- US-A1- 2007 141 133
- US-A1- 2008 113 900
- US-A1- 2008 145 449
- US-B1- 6 312 734
- JIN T ET AL: "Gd<3+>-functionalized near-infrared quantum dots for in vivo dual modal (fluorescence/magnetic resonance) imaging" CHEMICAL COMMUNICATIONS 2008 GB LNKD- DOI:10.1039/B812302K, no. 44, 2008, pages 5764-5766, XP002590811 ISSN: 1359-7345
- JIN TAKASHI ET AL: "Preparation and characterization of highly fluorescent, glutathione-coated near infrared quantum dots for in vivo fluorescence imaging." INTERNATIONAL JOURNAL OF MOLECULAR SCIENCES OCT 2008 LNKD- PUBMED:19325735, vol. 9, no. 10, October 2008 (2008-10), pages 2044-2061, XP002590998 ISSN: 1422-0067
- LANIUS R A ET AL: "Increased [<35>S]glutathione binding sites in spinal cords from patients with sporadic amyotrophic lateral sclerosis" NEUROSCIENCE LETTERS, LIMERICK, IE LNKD- DOI:10.1016/0304-3940(93)90236-E, vol. 163, no. 1, 26 November 1993 (1993-11-26), pages 89-92, XP024374214 ISSN: 0304-3940 [retrieved on 1993-11-26]
- ZLOKOVIC B V: "Cerebrovascular permeability to peptides: Manipulations of transport systems at the blood-brain barrier" PHARMACEUTICAL RESEARCH 1995 US LNKD- DOI:10.1023/A:1016254514167, vol. 12, no. 10, 1995, pages 1395-1406, XP008124101 ISSN: 0724-8741
- DEDEOGLU A ET AL: "Magnetic resonance spectroscopic analysis of Alzheimer's disease mouse brain that express mutant human APP shows altered neurochemical profile" BRAIN RESEARCH 20040625 NL LNKD- DOI:10.1016/J.BRAINRES.2004.02.079, vol. 1012, no. 1-2, 25 June 2004 (2004-06-25), pages 60-65, XP002590830 ISSN: 0006-8993
- DE BOER ALBERTUS G ET AL: "Strategies to improve drug delivery across the blood-brain barrier." CLINICAL PHARMACOKINETICS 2007 LNKD- PUBMED:17596102, vol. 46, no. 7, 2007, pages 553-576, XP008124054 ISSN: 0312-5963
- BURG D ET AL: "GLUTATHIONE CONJUGATES AND THEIR SYNTHETIC DERIVATIVES AS INHIBITORS OF GLUTATHIONE-DEPENDENT ENZYMES INVOLVED IN CANCER AND DRUG RESISTANCE" DRUG METABOLISM REVIEWS, MARCEL DEKKER, NEW YORK, NY, US LNKD- DOI:10.1081/DMR-120015695, vol. 34, no. 4, 1 January 2002 (2002-01-01), pages 821-863, XP008045263 ISSN: 0360-2532
- MORE S S ET AL: "Design, synthesis and biological evaluation of glutathione peptidomimetics as components of anti-Parkinson prodrugs" JOURNAL OF MEDICINAL CHEMISTRY 20080814 US LNKD- DOI:10.1021/JM800239V, vol. 51, no. 15, 14 August 2008 (2008-08-14), pages 4581-4588, XP002602082 ISSN: 0022-2623 cited in the application
- PRIEBE W ET AL: "Doxorubicin- and daunorubicin-glutathione conjugates, but not unconjugated drugs, competitively inhibit leukotriene C4 transport mediated by MRP/GS-X pump" BIOCHEMICAL AND BIOPHYSICAL RESEARCH COMMUNICATIONS 19980629 US LNKD- DOI:10.1006/BBRC.1998.8887, vol. 247, no. 3, 29 June 1998 (1998-06-29) , pages 859-863, XP005300992 ISSN: 0006-291X
- Rip J. et al: "Receptor-mediated delivery of drugs across the blood-brain barrier" Conference: Pharmacology and Toxicology of the Blood-Barin Barrier: State of the Art, Needs for future Research and Expected Benefits for the EU, Belgium February 11-12, 2010 11 March 2010 (2010-03-11), XP002602084 Retrieved from the Internet: URL:http://www.frontiersin.org/Community/A bstractDetails.aspx?ABS_DOI=10.3389/conf.f phar.2010.02.00025 [retrieved on 2010-09-24]
- FABEL KLAUS ET AL: "Long-term stabilization in patients with malignant glioma after treatment with liposomal doxorubicin", CANCER, vol. 92, no. 7, 1 October 2001 (2001-10-01), pages 1936-1942, XP055493092, ISSN: 0008-543X

## Description

### Field of the invention

This invention relates to the field of targeted drug delivery. The invention relates to conjugates of active pharmaceutical ingredients, comprised in carriers or nanocontainers, linked with ligands for glutathione transporters that mediate specific binding, endo- or transcytosis. These conjugates are preferably used in methods for treatment or prevention of brain-based conditions.

### Background of the invention

In order to function properly, neurons require a tightly regulated extracellular milieu. This essential, well-defined microenvironment is locally maintained by nursing brain cells called astrocytes (or astroglia). To cope with the considerable and variable dissimilarity between the composition of the blood and the extracellular compartment of the brain, the central nervous system (CNS) is also shielded from the general blood circulation by a number of blood-CNS barriers, i.e. the blood-brain barrier, blood-cerebral spinal fluid (CSF) barrier, pial vessel-CSF barrier, the ependyma and glia limitans, and also the blood-retina barrier, blood-nerve barrier, blood-spinal cord barrier. The blood-brain barrier (BBB) is considered as the most important blood-CNS barrier, because it covers a 1000 times larger surface area when compared to the other blood-CNS barriers. The BBB is characterised by a unique tight endothelial cell layer that covers capillary blood vessels in the CNS. Again, astrocytes are the principal inducers of BBB properties in these endothelial cells, by projecting 'glialfoot' on the capillaries.

In particular, the BBB regulates the trafficking of ions (Na+, K+, Ca2+), water, nutrients, metabolites, neurotransmitters (glutamic acid, tryptophan), plasma proteins (albumin, fibrinogen, immunoglobulins), cells from the immune system and also xenobiotics (drugs) in and out of the brain. The capillary endothelium in the brain has special properties when compared to peripheral capillaries. It has narrow tight-junctions, no fenestrae, low pinocytotic activity and a continuous basement membrane. The narrow tight-junctions result in a high electrical resistance of 1500-2000 Ohm.cm2. In addition, the endothelial cells have a negative surface charge that repulses negatively charged compounds. They have many mitochondria and enzymes to break down compounds and various selective transport systems to actively transport nutrients and other compounds into and out of the brain. In general, the BBB can be regarded as an organ that serves to protect the homeostasis of the brain. The BBB, however, thus also limits the delivery of xenobiotics (such as drugs and diagnostic agents) to the brain, which complicates classical drug therapy (i.e. targeted against neurons) of brain disorders. It is therefore desirable to selectively target drugs to the brain via endogenous BBB transport systems.

In addition, for some classes of drugs to reach their intracellular targets, they need to be delivered across the lipophilic cell membrane, into the hydrophilic cytoplasm. This lipophilic to hydrophilic transport requirement forms a challenge for the design and delivery of many of such drugs. Administering drugs in a non-phosphorylated form, may improve cell entrance of a drug, since the cell membrane is poorly permeable to phosphorylated drugs. Subsequently, the drug may be phosphorylated into its active form by a thymidine kinase. However, drug-uptake will occur non-specifically by all body tissues. Another drawback is that, it may take up to 4 weeks of dosing to achieve steady state plasma levels of the drug. Some treatments require for instance daily administration of high doses (800-1200 mg/day) for periods of 24-48 weeks. This is too late for the treatment of non-chronic conditions. Yet another drawback is that such treatments are usually limited by toxicity. In conclusion, for many treatments and therapies there is a need for the delivery of an effective amount of drugs in a suitable time period to a desired site while minimizing side effects. Perhaps the biggest challenge lies in the (timely) delivery of drugs to sites protected by physiological barriers, such as the CNS, retina, placenta and testes.

There are no CNS drugs on the market yet that target specific uptake receptors. A large portion of the marketed drugs for the treatment of neurological disorders (like stroke, migraine and MS), are in fact directed against targets outside the brain (e.g., cerebral vasculature, or immune system). Unlike small molecules, biopharmaceutical drugs are unlikely candidates for chemical modifications to enhance their permeability across the blood-brain barrier. Such compounds now rely on invasive and harmful technologies to patients, like direct and local stereotactic injections, intrathecal infusions and even (pharmacological) disruption of the blood-brain barrier. Because of the severe neurological consequences of these techniques, these are only warranted in selected life-threatening diseases. Moreover, local administrations are far from effective in delivering drugs throughout the large human brain. Innovative CNS drug delivery technologies are thus highly awaited.

Glutathione (GSH) is an endogenous antioxidant. If concentration thereof in serum is insufficient, some nervous diseases, such as chronic fatigue syndrome (CFS), may occur. In 1994, Berislav V. Zlokovic asserted that GSH reaches and passes through the BBB of a guinea pig via a special route, such as GSH-transporter, without decomposition (1994, Biochem. Biophys. Res. Commun. 201: 402-408). In 1995, Berislav V. Zlokovic asserted that GSH exists in brain, astrocyte and endothelial cells in millimolar concentration (1995, Pharm. Res. 12: 1395-1406). In 1995, Ram Kannan asserted that GSH uptake depends on Na+ concentration (1995, Invest. Ophthalmol. Vis. Sci. 36: 1785-1792). If Na+ concentration is low, GSH uptake from brain endothelial cells may be inhibited. He also pointed Na-dependent GSH transporter located on the luminal side of the BBB manages GSH uptake and Na-independent GSH transporter located on the luminal side of the BBB manages efflux of GSH (1996, J. Biol. Chem. 271: 9754-9758). Additionally, Kannan constructed a rat hepatic canalicular GSH transporter (RcGSHT) system using the brains of mice and guinea pigs to analyze cDNA fragments 5, 7, and 11. The results indicate that fragment 7 represents Na-dependent GSH transporter and fragments 5 and 11 represent Na-independent GSH transporter. In 1999, Ram Kannan built a mouse brain endothelial cell line (MBEC-4) model simulating BBB situation (1999, J. Neurochem. 73: 390-399). The model proved that Na-dependent GSH transporter is located on the luminal side of the MBEC-4 cell. In 2000, Ram Kannan asserted that GSH passes through the BBB via Na-dependent GSH transporter in human cerebrovascular endothelial cells (HCBC) and Na-dependent GSH transporter exists in the luminal plasma membrane of HCEC (2000, Brain. Res. 852: 374-382). In 2003, Zhao Zhiyang provided an anticancer pro-drug bonded with glutathione s-transferase (GST)/glutathione (GSH) by sulfonamide covalent bonds to target and treat specific cancer cells after break of the sulfonamide bonds recited in US2003109555. This modification can protect amino groups of drugs, increase solubility thereof, and alter absorption and distribution thereof in body. In 2005, Ae-June Wang et al., disclosed in U.S. Pat. No. 7,446,096 an invention providing a delivery system comprising a carrier or an active compound and a glutathione or a glutathione derivative grafted thereon. The invention also provides a compound comprising a moiety comprising a vitamin E derivative or a phospholipid derivative, a polyethylene glycol (PEG) or a polyethylene glycol derivative bonded thereto, and a glutathione (GSH) or a glutathione derivative bonded to the polyethylene glycol or the PEG derivative. In 2008, Pieter Gaillard disclosed the (CNS) targeted delivery of antiviral chemotherapeutics and other antiviral agents in U. S. Pat. Application No 60/907,176 using GSH-PEG liposomes both *in vitro* and *in vivo.* Later that year, Swati More and Robert Vince published a paper on the design, synthesis and biological evaluation of glutathione peptidomimetics as components of anti-Parkinson prodrugs using *in vitro* methods (More, 2008, J.Med. Chem. 51: 4581-4588). Fabel K. et al. (Cancer, 2001, vol. 92, no. 7, p. 1936-1942) described the long-term stabilization in patients with malignant glioma after treatment with liposomal doxorubicin.

These disclosures do however not provide sufficient detail for specific and relevant combinations between drugs and compounds that have specific use for the diagnosis and/or (preventive) treatment of specific CNS and related disorders. It is therefore an object of the invention to provide for a safe, effective and versatile approach for carrying cargo such as small molecules, peptides, proteins and nanocontaines (such as liposomes) containing drugs and genes across the cell membrane and across a blood-tissue barrier such as the blood-brain barrier for *inter alia* CNS drug targeting using glutathione transport receptors. Surprisingly, we established that in addition to the disclosures described above, conjugation of glutathione and glutathione derivatives to peptides, proteins (like enzymes and antibodies), other small molecules, and polyplexes, either by direct coupling or linking by using spacer molecules, is effective in targeting agents specifically to glutathione transport receptors.

### Description of the invention

### Definitions

The terms "oligonucleotide" and "polynucleotide" as used herein include linear oligo- and polymers of natural or modified monomers or linkages, including deoxyribonucleosides, ribonucleosides, α-anomeric forms thereof, polyamide nucleic acids, and the like, capable of specifically binding to a target polynucleotide by way of a regular pattern of monomer-to-monomer interactions (e.g. nucleoside-to-nucleoside), such as Watson-Crick type of base pairing, Hoogsteen or reverse Hoogsteen types of base pairing, or the like. Usually monomers are linked by phosphodiester bonds or analogs thereof to form oligonucleotides ranging in size from a few monomeric units, e.g. 3-4, to several hundreds of monomeric units. Whenever an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxyguanosine, and "T" denotes thymidine, unless otherwise noted. Analogs of phosphodiester linkages include phosphorothioate, phosphorodithioate, phosphoroselenoate, phosphorodiselenoate, phosphoroanilothioate, phosphoranilidate, phosphoramidate, N3'→P5' phosphoramidate and the like. A polynucleotide can be of substantially any length, typically from about 10 nucleotide to about 1x10⁹ nucleotide or larger. As used herein, an "oligonucleotide" is defined as a polynucleotide of from 4 to 100 nucleotide in length. Thus, an oligonucleotide is a subset of polynucleotides.

As used herein, the term "specific binding" means binding that is measurably different from a non-specific interaction. Specific binding can be measured, for example, by determining binding of a molecule (ligand) compared to binding of a control molecule (ligand), which generally is a molecule of similar structure that does not have binding activity, for example, a peptide of similar size that lacks a specific binding sequence. Specific binding is present if a ligand has measurably higher affinity for the receptor than the control ligand. Specificity of binding can be determined, for example, by competition with a control ligand that is known to bind to a target. The term "specific binding," as used herein, includes both low and high affinity specific binding. Specific binding can be exhibited, e.g., by a low affinity targeting agent having a Kd of at least about 10⁻⁴ M. E.g., if a receptor has more than one binding site for a ligand, a ligand having low affinity can be useful for targeting the microvascular endothelium. Specific binding also can be exhibited by a high affinity ligands, e.g. a ligand having a Kd of at least about of 10⁻⁷ M, at least about 10⁻⁸ M, at least about 10⁻⁹ M, at least about 10⁻¹⁰ M, or can have a Kd of at least about 10⁻¹¹ M or 10⁻¹² M or greater. Both low and high affinity-targeting ligands are useful for incorporation in the conjugates of the present invention.

### Detailed description of the invention

The present invention is based on a safe, endogenous (non-toxic) transport mechanism, called receptor-mediated endocytosis, for carrying therapeutic moieties, such as large proteins and liposomes containing drugs and genes, i.e. drugs and genes encapsulated in liposomes, across a cell membrane or across a blood-tissue barrier such as the blood-brain barrier for e.g. brain delivery thereof. A range of validated and well known internalizing receptors are present at cells and the blood-brain barrier for this use. These include, but are not limited to, the thiamine transporter, alpha(2,3)-sialoglycoprotein receptor, transferrin receptor, scavenger receptors, LDL receptors, LRP1B, LRP2, DTR, insulin receptor, IGF receptor, leptin receptor, mannose 6-phosphate receptor. The present invention however relates to a safer and more effective way of specifically delivering, or specifically enhancing the delivery of, drugs to cells and across the blood-brain barrier by targeting to endogenous internalizing uptake (transport) receptors for glutathione on the capillaries in the brain, without modifying or disrupting the normal function of the neuroprotective blood-brain barrier.

In a first aspect the present disclosure relates to a conjugate comprising: a) a ligand for a glutathione transporter; and, b) at least one of a diagnostic or therapeutic agent, and a pharmaceutically acceptable nanocontainer comprising the agent; wherein the ligand in a) preferably is conjugated to at least one of the agent and nanocontainer in b). The invention relates to a conjugate comprising: a) a ligand for a glutathione transporter; wherein the ligand is selected from the group consisting of: glutathione, S-(p-bromobenzyl)glutathione, gamma-(L-gamma-azaglutamyl)-S-(p-bromobenzyl)-L-cysteinylglycin, S-butylglutathione, S-decylglutathione, glutathione reduced ethyl ester, glutathionesulfonic acid, S-hexylglutathione, S-lactoylglutathione, S-methylglutathione, S-(4-nitrobenzyl)glutathione, S-octylglutathione, S-propylglutathione, n-butanoyl gamma-glutamylcysteinylglycine, ethanoyl gamma-glutamylcysteinylglycine, hexanoyl gamma-glutamylcysteinylglycine, octanoyl gamma-glutamylcysteinylglycine, dodecanoyl gamma-glutamylcysteinylglycine, GSH monoisopropyl ester (N-(N-L-glutamyl-L-cysteinyl)glycine 1-isopropyl ester sulfate monohydrate) and glutathione derivatives of the formula I: 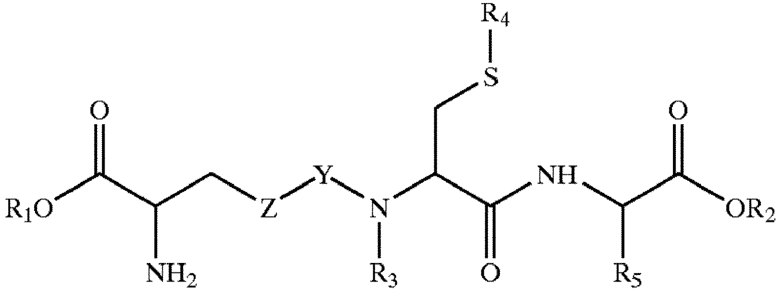
wherein Z=CH₂ and Y=CH₂, or Z=O and Y = C=O;
R₁ and R₂ are independently selected from the group consisting of:
   i) H,
   ii) linear or branched alkyl (1-25C),
   iii) aralkyl (6-26C),
   iv) cycloalkyl (6-25C),
   v) heterocycles (6-20C),
   vi) ethers or polyethers (3-25C), and
   vii) where R₁-R₂ together have 2-20C atoms and form a macrocycle with the remainder of formula I;
R₃ is selected from the group consisting of H and CH₃;
R4 is selected form the group consisting of 6-8C alkyl, benzyl, naphthyl and a therapeutically active compound; and,
R5 is selected from the group consisting of H, phenyl, CH₃- and CH₂-phenyl; or,
   a pharmaceutically acceptable salt thereof,
   and,
   b) a liposome comprising an anthracycline,
wherein the ligand in a) is conjugated to the liposome in b).

A "conjugate" is herein defined as consisting of two entities that are coupled together. Preferably, the two entities are conjugated by non-specific or specific protein-protein interaction, by covalent bonding, by non-covalent bonding, by coordinating chemical bonding, by chemical synthesis, either directly or via a (non)cleavable spacers, linkers or components of nanocontainers, or by recombinant technologies. In the context of the present disclosure the first entity may be the diagnostic and/or therapeutic agent or the pharmaceutically acceptable nanocontainer comprising the agent while in the context of the present invention the first entity is a liposome comprising an anthracycline, whereas the second entity will be a ligand for a glutathione transporter on a target cell. Suitable diagnostic and/or therapeutic agents, pharmaceutically acceptable nanocontainer for such agents, as well as suitable ligands for glutathione transporters for use in the conjugates of the invention are further defined herein below.

### Therapeutic or diagnostic agents

The conjugates of the invention comprise at least one agent, being an anthracycline. Anthracyclines are cytostatics or antineoplastic agents, and are cytotoxic/antitumor antibiotics (Aclarubicin, Daunorubicin, Doxorubicin, Epirubicin, Idarubicin, Amrubicin, Pirarubicin, Valrubicin, Zorubicin).

As part of the disclosure preferred agent for incorporation in the conjugates of the invention is a small molecule chemical agent. A chemical agent is herein understood to be a defined chemical molecule, usually a smaller, non-polymeric molecule (e.g. less than 2kDa) that is at least partially organic, that usually may be obtained by chemical synthesis and that does not comprise an oligo- or poly-nucleotide. Drug compounds or agents of interest from which small molecule drug moieties may be derived are also listed in: Goodman & Gilman's, The Pharmacological Basis of Therapeutics (9th Ed) (Goodman et al. eds) (McGraw-Hill) (1996); and 1999 Physician's Desk Reference (1998). Additional specific drugs and compounds of interest from which the drug moiety of the disclosure may be derived include, but are not limited to:
Central nervous system depressants: general anesthetics (barbiturates, benzodiazepines, steroids, cyclohexanone derivatives, and miscellaneous agents), sedative-hypnotics (benzodiazepines, barbiturates, piperidinediones and triones, quinazoline derivatives, carbamates, aldehydes and derivatives, amides, acyclic ureides, benzazepines and related drugs, phenothiazines, etc.), central voluntary muscle tone modifying drugs (anticonvulsants, such as hydantoins, barbiturates, oxazolidinediones, succinimides, acylureides, glutarimides, benzodiazepines, secondary and tertiary alcohols, dibenzazepine derivatives, valproic acid and derivatives, GABA analogs, etc.), analgesics (morphine and derivatives, oripavine derivatives, morphinan derivatives, phenylpiperidines, 2,6-methane-3-benzazocaine derivatives, diphenylpropylamines and isosteres, salicylates, p-aminophenol derivatives, 5-pyrazolone derivatives, arylacetic acid derivatives, fenamates and isosteres, naltrexone, methylnaltrexone, etc.) and antiemetics (anticholinergics, antihistamines, antidopaminergics, etc.), analgesic agents as disclosed in U. S. Pat. Nos: 5,292,736, 5,688,825, 5,554,789, 5,455,230, 5,292,736, 5,298,522, 5,216,165, 5,438,064, 5,204,365, 5,017,578, 4,906,655, 4,906,655, 4,994,450, 4,749,792, 4,980,365, 4,794,110, 4,670,541, 4,737,493, 4,622,326, 4,536,512, 4,719,231, 4,533,671, 4,552,866, 4,539,312, 4,569,942, 4,681,879, 4,511,724, 4,556,672, 4,721,712, 4,474,806, 4,595,686, 4,440,779, 4,434,175, 4,608,374, 4,395,402, 4,400,534, 4,374,139, 4,361,583, 4,252,816, 4,251,530, 5,874,459, 5,688,825, 5,554,789, 5,455,230, 5,438,064, 5,298,522, 5,216,165, 5,204,365, 5,030,639, 5,017,578, 5,008,264, 4,994,450, 4,980,365, 4,906,655, 4,847,290, 4,844,907, 4,794,110, 4,791,129, 4,774,256, 4,749,792, 4,737,493, 4,721,712, 4,719,231, 4,681,879, 4,670,541, 4,667,039, 4,658,037, 4,634,708, 4,623,648, 4,622,326, 4,608,374, 4,595,686, 4,594,188, 4,569,942, 4,556,672, 4,552,866, 4,539,312, 4,536,512, 4,533,671, 4,511,724, 4,440,779, 4,434,175, 4,400,534, 4,395,402, 4,391,827, 4,374,139, 4,361,583, 4,322,420, 4,306,097, 4,252,816, 4,251,530, 4,244,955, 4,232,018, 4,209,520, 4,164,514, 4,147,872, 4,133,819, 4,124,713, 4,117,012, 4,064,272, 4,022,836, 3,966,944;
Central nervous system stimulants: analeptics (respiratory stimulants, convulsant stimulants, psychomotor stimulants), narcotic antagonists (morphine derivatives, oripavine derivatives, 2,6-methane-3-benzoxacine derivatives, morphinan derivatives), nootropics, flumazenil;
Psychopharmacologicals: anxiolytic sedatives (benzodiazepines, propanediol carbamates) antipsychotics (phenothiazine derivatives, thioxanthine derivatives, other tricyclic compounds, butyrophenone derivatives and isosteres, diphenylbutylamine derivatives, substituted benzamides, arylpiperazine derivatives, indole derivatives, etc.), antidepressants (tricyclic compounds, MAO inhibitors, etc.), agents as disclosed in U. S. Pat. Nos: 5,192,799, 5,036,070, 4,778,800, 4,753,951, 4,590,180, 4,690,930, 4,645,773, 4,427,694, 4,424,202, 4,440,781, 5,686,482, 5,478,828, 5,461,062, 5,387,593, 5,387,586, 5,256,664, 5,192,799, 5,120,733, 5,036,070, 4,977,167, 4,904,663, 4,788,188, 4,778,800, 4,753,951, 4,690,930, 4,645,773, 4,631,285, 4,617,314, 4,613,600, 4,590,180, 4,560,684, 4,548,938, 4,529,727, 4,459,306, 4,443,451, 4,440,781, 4,427,694, 4,424,202, 4,397,853, 4,358,451, 4,324,787, 4,314,081, 4,313,896, 4,294,828, 4,277,476, 4,267,328, 4,264,499, 4,231,930, 4,194,009, 4,188,388, 4,148,796, 4,128,717, 4,062,858, 4,031,226, 4,020,072, 4,018,895, 4,018,779, 4,013,672, 3,994,898, 3,968,125, 3,939,152, 3,928,356, 3,880,834, 3,668,210;
Respiratory tract drugs: central antitussives (opium alkaloids and their derivatives);
Peripheral nervous system drugs: local anesthetics (ester derivatives, amide derivatives);
Drugs acting at synaptic or neuroeffector junctional sites: cholinergic agents, cholinergic blocking agents, neuromuscular blocking agents, adrenergic agents, antiadrenergic agents, cholinergic agents as disclosed in U. S. Pat. Nos: 5,219,872, 5,219,873, 5,073,560, 5,073,560, 5,346,911, 5,424,301, 5,073,560, 5,219,872, 4,900,748, 4,786,648, 4,798,841, 4,782,071, 4,710,508, 5,482,938, 5,464,842, 5,378,723, 5,346,911, 5,318,978, 5,219,873, 5,219,872, 5,084,281, 5,073,560, 5,002,955, 4,988,710, 4,900,748, 4,798,841, 4,786,648, 4,782,071, 4,745,123, 4,710,508; adrenergic agents as disclosed in U. S. Pat. Nos: 5,091,528, 5,091,528, 4,835,157, 5,708,015, 5,594,027, 5,580,892, 5,576,332, 5,510,376, 5,482,961, 5,334,601, 5,202,347, 5,135,926, 5,116,867, 5,091,528, 5,017,618, 4,835,157, 4,829,086, 4,579,867, 4,568,679, 4,469,690, 4,395,559, 4,381,309, 4,363,808, 4,343,800, 4,329,289, 4,314,943, 4,311,708, 4,304,721, 4,296,117, 4,285,873, 4,281,189, 4,278,608, 4,247,710, 4,145,550, 4,145,425, 4,139,535, 4,082,843, 4,011,321, 4,001,421, 3,982,010, 3,940,407, 3,852,468, 3,832,470;
Smooth muscle active drugs: spasmolytics (anticholinergics, musculotropic spasmolytics), vasodilators, smooth muscle stimulants;
Histamines and antihistamines: histamine and derivative thereof (betazole), antihistamines (H1-antagonists, H2-antagonists), histamine metabolism drugs, agents as disclosed in U. S. Pat. Nos: 5,874,479, 5,863,938, 5,856,364, 5,770,612, 5,702,688, 5,674,912, 5,663,208, 5,658,957, 5,652,274, 5,648,380, 5,646,190, 5,641,814, 5,633,285, 5,614,561, 5,602,183, 4,923,892, 4,782,058, 4,393,210, 4,180,583, 3,965,257, 3,946,022, 3,931,197;
Cardiovascular drugs: cardiotonics (plant extracts, butenolides, pentadienolids, alkaloids from erythrophleum species, ionophores, adrenoceptor stimulants, etc), antiarrhythmic drugs, antihypertensive agents, antilipidemic agents (clofibric acid derivatives, nicotinic acid derivatives, hormones and analogs, antibiotics, salicylic acid and derivatives), antivaricose drugs, hemostyptics, agents as disclosed in U. S. Pat. Nos: 4,966,967, 5,661,129, 5,552,411, 5,332,737, 5,389,675, 5,198,449, 5,079,247, 4,966,967, 4,874,760, 4,954,526, 5,051,423, 4,888,335, 4,853,391, 4,906,634, 4,775,757, 4,727,072, 4,542,160, 4,522,949, 4,524,151, 4,525,479, 4,474,804, 4,520,026, 4,520,026, 5,869,478, 5,859,239, 5,837,702, 5,807,889, 5,731,322, 5,726,171, 5,723,457, 5,705,523, 5,696,111, 5,691,332, 5,679,672, 5,661,129, 5,654,294, 5,646,276, 5,637,586, 5,631,251, 5,612,370, 5,612,323, 5,574,037, 5,563,170, 5,552,411, 5,552,397, 5,547,966, 5,482,925, 5,457,118, 5,414,017, 5,414,013, 5,401,758, 5,393,771, 5,362,902, 5,332,737, 5,310,731, 5,260,444, 5,223,516, 5,217,958, 5,208,245, 5,202,330, 5,198,449, 5,189,036, 5,185,362, 5,140,031, 5,128,349, 5,116,861, 5,079,247, 5,070,099, 5,061,813, 5,055,466, 5,051,423, 5,036,065, 5,026,712, 5,011,931, 5,006,542, 4,981,843, 4,977,144, 4,971,984, 4,966,967, 4,959,383, 4,954,526, 4,952,692, 4,939,137, 4,906,634, 4,889,866, 4,888,335, 4,883,872, 4,883,811, 4,847,379, 4,835,157, 4,824,831, 4,780,538, 4,775,757, 4,774,239, 4,771,047, 4,769,371, 4,767,756, 4,762,837, 4,753,946, 4,752,616, 4,749,715, 4,738,978, 4,735,962, 4,734,426, 4,734,425, 4,734,424, 4,730,052, 4,727,072, 4,721,796, 4,707,550, 4,704,382, 4,703,120, 4,681,970, 4,681,882, 4,670,560, 4,670,453, 4,668,787, 4,663,337, 4,663,336, 4,661,506, 4,656,267, 4,656,185, 4,654,357, 4,654,356, 4,654,355, 4,654,335, 4,652,578, 4,652,576, 4,650,874, 4,650,797, 4,649,139, 4,647,585, 4,647,573, 4,647,565, 4,647,561, 4,645,836, 4,639,461, 4,638,012, 4,638,011, 4,632,931, 4,631,283, 4,628,095, 4,626,548, 4,614,825, 4,611,007, 4,611,006, 4,611,005, 4,609,671, 4,608,386, 4,607,049, 4,607,048, 4,595,692, 4,593,042, 4,593,029, 4,591,603, 4,588,743, 4,588,742, 4,588,741, 4,582,854, 4,575,512, 4,568,762, 4,560,698, 4,556,739, 4,556,675, 4,555,571, 4,555,570, 4,555,523, 4,550,120, 4,542,160, 4,542,157, 4,542,156, 4,542,155, 4,542,151, 4,537,981, 4,537,904, 4,536,514, 4,536,513, 4,533,673, 4,526,901, 4,526,900, 4,525,479, 4,524,151, 4,522,949, 4,521,539, 4,520,026, 4,517,188, 4,482,562, 4,474,804, 4,474,803, 4,472,411, 4,466,979, 4,463,015, 4,456,617, 4,456,616, 4,456,615, 4,418,076, 4,416,896, 4,252,815, 4,220,594, 4,190,587, 4,177,280, 4,164,586, 4,151,297, 4,145,443, 4,143,054, 4,123,550, 4,083,968, 4,076,834, 4,064,259, 4,064,258, 4,064,257, 4,058,620, 4,001,421, 3,993,639, 3,991,057, 3,982,010, 3,980,652, 3,968,117, 3,959,296, 3,951,950, 3,933,834, 3,925,369, 3,923,818, 3,898,210, 3,897,442, 3,897,441, 3,886,157, 3,883,540, 3,873,715, 3,867,383, 3,873,715, 3,867,383, 3,691,216, 3,624,126;
Gastrointestinal tract drugs: digestants (stomachics, choleretics), antiulcer drugs, antidiarrheal agents;
Steroidal agents: Hydrocortisone (cortisol), cortisone acetate, prednisone, prednisolone, methylprednisolone or methylprednisolone acetate or methylprednisolone hemisuccinate, dexamethasone, betamethasone, triamcinolone, beclometasone, fludrocortisone acetate or hemisuccinate, deoxycorticosterone acetate (DOCA) or hemicussinate, aldosterone, including as disclosed in U. S. Pat. Nos: 5,863,538, 5,855,907, 5,855,866, 5,780,592, 5,776,427, 5,651,987, 5,346,887, 5,256,408, 5,252,319, 5,209,926, 4,996,335, 4,927,807, 4,910,192, 4,710,495, 4,049,805, 4,004,005, 3,670,079, 3,608,076, 5,892,028, 5,888,995, 5,883,087, 5,880,115, 5,869,475, 5,866,558, 5,861,390, 5,861,388, 5,854,235, 5,837,698, 5,834,452, 5,830,886, 5,792,758, 5,792,757, 5,763,361, 5,744,462, 5,741,787, 5,741,786, 5,733,899, 5,731,345, 5,723,638, 5,721,226, 5,712,264, 5,712,263, 5,710,144, 5,707,984, 5,705,494, 5,700,793, 5,698,720, 5,698,545, 5,696,106, 5,677,293, 5,674,861, 5,661,141, 5,656,621, 5,646,136, 5,637,691, 5,616,574, 5,614,514, 5,604,215, 5,604,213, 5,599,807, 5,585,482, 5,565,588, 5,563,259, 5,563,131, 5,561,124, 5,556,845, 5,547,949, 5,536,714, 5,527,806, 5,506,354, 5,506,221, 5,494,907, 5,491,136, 5,478,956, 5,426,179, 5,422,262, 5,391,776, 5,382,661, 5,380,841, 5,380,840, 5,380,839, 5,373,095, 5,371,078, 5,352,809, 5,344,827, 5,344,826, 5,338,837, 5,336,686, 5,292,906, 5,292,878, 5,281,587, 5,272,140, 5,244,886, 5,236,912, 5,232,915, 5,219,879, 5,218,109, 5,215,972, 5,212,166, 5,206,415, 5,194,602, 5,166,201, 5,166,055, 5,126,488, 5,116,829, 5,108,996, 5,099,037, 5,096,892, 5,093,502, 5,086,047, 5,084,450, 5,082,835, 5,081,114, 5,053,404, 5,041,433, 5,041,432, 5,034,548, 5,032,586, 5,026,882, 4,996,335, 4,975,537, 4,970,205, 4,954,446, 4,950,428, 4,946,834, 4,937,237, 4,921,846, 4,920,099, 4,910,226, 4,900,725, 4,892,867, 4,888,336, 4,885,280, 4,882,322, 4,882,319, 4,882,315, 4,874,855, 4,868,167, 4,865,767, 4,861,875, 4,861,765, 4,861,763, 4,847,014, 4,774,236, 4,753,932, 4,711,856, 4,710,495, 4,701,450, 4,701,449, 4,689,410, 4,680,290, 4,670,551, 4,664,850, 4,659,516, 4,647,410, 4,634,695, 4,634,693, 4,588,530, 4,567,000, 4,560,557, 4,558,041, 4,552,871, 4,552,868, 4,541,956, 4,519,946, 4,515,787, 4,512,986, 4,502,989, 4,495,102;
Cytostatics or antineoplastic agents: Antimetabolites: Folic acid (Aminopterin, Methotrexate, Pemetrexed, Raltitrexed), Purine (Cladribine, Clofarabine, Fludarabine, Mercaptopurine, Pentostatin, Thioguanine), Pyrimidine (Cytarabine, Decitabine, Fluorouracil/Capecitabine, Floxuridine, Gemcitabine, Enocitabine, Sapacitabine); Alkylating/alkylating-like: Nitrogen mustards (Chlorambucil, Chlormethine, Cyclophosphamide, Ifosfamide, Melphalan, Bendamustine, Trofosfamide, Uramustine), Nitrosoureas (Carmustine, Fotemustine, Lomustine, Nimustine, Prednimustine, Ranimustine, Semustine, Streptozocin), Platinum (alkylating-like) (Carboplatin, Cisplatin, Nedaplatin, Oxaliplatin, Triplatin tetranitrate, Satraplatin), Alkyl sulfonates (Busulfan, Mannosulfan, Treosulfan), Hydrazines (Procarbazine), Triazenes (Dacarbazine, Temozolomide), Aziridines (Carboquone, ThioTEPA, Triaziquone, Triethylenemelamine); Spindle poisons/mitotic inhibitors: Taxanes (Docetaxel, Larotaxel, Ortataxel, Paclitaxel, Tesetaxel), and Vinca alkaloids (Vinblastine, Vincristine, Vinflunine, Vindesine, Vinorelbine), Ixabepilone; Cytotoxic/antitumor antibiotics: Anthracenediones (Mitoxantrone, Pixantrone), Streptomyces (Actinomycin, Bleomycin, Mitomycin, Plicamycin), Hydroxyurea; Topoisomerase inhibitors: Camptotheca (Camptothecin, Topotecan, Irinotecan, Rubitecan, Belotecan), Podophyllum (Etoposide, Teniposide); Tyrosine kinase inhibitors: Axitinib, Bosutinib, Cediranib, Dasatinib, Erlotinib, Gefitinib, Imatinib, Lapatinib, Lestaurtinib, Neratinib, Nilotinib, Semaxanib, Sorafenib, Sunitinib, Vandetanib; Cyclin-dependent kinase inhibitors: Alvocidib, Seliciclib; Photosensitizers: Aminolevulinic acid/Methyl aminolevulinate, Efaproxiral, Porphyrin derivatives (Porfimer sodium, Talaporfin, Temoporfin, Verteporfin); Other: Altretamine, Amsacrine, Bexarotene, Estramustine, Irofulven, Trabectedin, Fusion protein (Aflibercept), Denileukin diftitox, Retinoids (Alitretinoin, Tretinoin), Anagrelide, Arsenic trioxide, Asparaginase/Pegaspargase, Atrasentan, Bortezomib, Carmofur, Celecoxib, Demecolcine, Elesclomol, Elsamitrucin, Etoglucid, Lonidamine, Lucanthone, Masoprocol, Mitobronitol, Mitoguazone, Mitotane, Oblimersen, Tegafur, Testolactone, Tiazofurine, Tipifarnib, Vorinostat; and agents as disclosed in U. S. Pat. Nos: 5,880,161, 5,877,206, 5,786,344, 5,760,041, 5,753,668, 5,698,529, 5,684,004, 5,665,715, 5,654,484, 5,624,924, 5,618,813, 5,610,292, 5,597,831, 5,530,026, 5,525,633, 5,525,606, 5,512,678, 5,508,277, 5,463,181, 5,409,893, 5,358,952, 5,318,965, 5,223,503, 5,214,068, 5,196,424, 5,109,024, 5,106,996, 5,101,072, 5,077,404, 5,071,848, 5,066,493, 5,019,390, 4,996,229, 4,996,206, 4,970,318, 4,968,800, 4,962,114, 4,927,828, 4,892,887, 4,889,859, 4,886,790, 4,882,334, 4,882,333, 4,871,746, 4,863,955, 4,849,563, 4,845,216, 4,833,145, 4,824,955, 4,785,085, 4,684,747, 4,618,685, 4,611,066, 4,550,187, 4,550,186, 4,544,501, 4,541,956, 4,532,327, 4,490,540, 4,399,283, 4,391,982, 4,383,994, 4,294,763, 4,283,394, 4,246,411, 4,214,089, 4,150,231, 4,147,798, 4,056,673, 4,029,661, 4,012,448;
Anti-infective agents: ectoparasiticides (chlorinated hydrocarbons, pyrethins, sulfurated compounds), anthelmintics, antiprotozoal agents, antimalarial agents, antiamebic agents, antileiscmanial drugs, antitrichomonal agents, antitrypanosomal agents, sulfonamides, antimycobacterial drugs, antiviral chemotherapeutics and other antiviral agents as disclosed in U. S. Pat. Application No: 60/907,176;
Antibiotics: aminoglycosides, e.g., amikacin, apramycin, arbekacin, bambermycins, butirosin, dibekacin, dihydrostreptomycin, fortimicin, gentamicin, isepamicin, kanamycin, micronomcin, neomycin, netilmicin, paromycin, ribostamycin, sisomicin, spectinomycin, streptomycin, tobramycin, trospectomycin; amphenicols, e.g., azidamfenicol, chloramphenicol, florfenicol, and theimaphenicol; ansamycins, e.g., rifamide, rifampin, rifamycin, rifapentine, rifaximin; beta lactams, e.g., carbacephems, carbapenems, cephalosporins, cehpamycins, monobactams, oxaphems, penicillins; lincosamides, e.g., clinamycin, lincomycin; macrolides, e.g., clarithromycin, dirthromycin, erythromycin, etc.; polypeptides, e.g. , amphomycin, bacitracin, capreomycin, etc.; tetracyclines, e.g., apicycline, chlortetracycline, clomocycline, etc.; synthetic antibacterial agents, such as 2,4diaminopyrimidines, nitrofurans, quinolones and analogs thereof, sulfonamides, sulfones;
Antifungal agents: polyenes, e.g., amphotericin B, candicidin, dermostatin, filipin, fungichromin, hachimycin, hamycin, lucensomycin, mepartricin, natamycin, nystatin, pecilocin, perimycin; synthetic antifungals, such as allylamines, e.g., butenafine, naftifine, terbinafine; imidazoles, e.g., bifonazole, butoconazole, chlordantoin, chlormidazole, etc.; thiocarbamates, e.g., tolciclate, triazole, e.g., fluconazole, itraconazole, terconazole;
Anthelmintics: arecoline, aspidin, aspidinol, dichlorophene, embelin, kosin, napthalene, niclosamide, pelletierine, quinacrine, alantolactone, amocarzine, amoscanate, ascaridole, bephenium, bitoscanate, carbon tetrachloride, carvacrol, cyclobendazole, diethylcarbamazine, etc;
Antimalarials: acedapsone, amodiaquin, arteether, artemether, artemisinin, artesunate, atovaquone, bebeerine, berberine, chirata, chlorguanide, chloroquine, chlorprogaunil, cinchona, cinchonidine, cinchonine, cycloguanil, gentiopicrin, halofantrine, hydroxychloroquine, mefloquine hydrochloride, 3-methylarsacetin, pamaquine, plasmocid, primaquine, pyrimethamine, quinacrine, quinine, quinine, quinocide, quinine, dibasic sodium arsenate;
Antiprotozoan agents: acranil, tinidazole, ipronidazole, ethylstibamine, pentamidine, acetarsone, aminitrozole, anisomycin, nifuratel, tinidazole, benzidazole, suramin, and the like;
Antimicrobial agents: As disclosed in U. S. Pat. Nos: 5,902,594, 5,874,476, 5,874,436, 5,859,027, 5,856,320, 5,854,242, 5,811,091, 5,786,350, 5,783,177, 5,773,469, 5,762,919, 5,753,715, 5,741,526, 5,709,870, 5,707,990, 5,696,117, 5,684,042, 5,683,709, 5,656,591, 5,643,971, 5,643,950, 5,610,196, 5,608,056, 5,604,262, 5,595,742, 5,576,341, 5,554,373, 5,541,233, 5,534,546, 5,534,508, 5,514,715, 5,508,417, 5,464,832, 5,428,073, 5,428,016, 5,424,396, 5,399,553, 5,391,544, 5,385,902, 5,359,066, 5,356,803, 5,354,862, 5, 346,913, 5,302,592, 5,288,693, 5,266,567, 5,254,685, 5,252,745, 5,209,930, 5,196,441, 5,190,961, 5,175,160, 5,157,051, 5,096,700, 5,093,342, 5,089,251, 5,073,570, 5,061,702, 5,037,809, 5,036,077, 5,010,109, 4,970,226, 4,916,156, 4,888,434, 4,870,093, 4,855,318, 4,784, 991, 4,746,504, 4,686,221, 4,599,228, 4,552,882, 4,492,700, 4,489,098, 4,489,085, 4,487,776, 4,479,953, 4,477,448, 4,474,807, 4,470,994, 4,370,484, 4,337,199, 4,311,709, 4,308,283, 4,304,910, 4,260,634, 4,233,311, 4,215,131, 4,166,122, 4,141,981, 4,130,664, 4,089,977, 4,089,900, 4,069,341, 4,055,655, 4,049,665, 4,044,139, 4,002,775, 3,991,201, 3,966,968, 3,954,868, 3,936,393, 3,917,476, 3,915,889, 3,867,548, 3,865,748, 3,867,548, 3,865,748, 3,783,160, 3,764,676, 3,764,677;
Anti-inflammatory agents: As disclosed in U. S. Pat. Nos: 5,872,109, 5,837,735, 5,827,837, 5,821,250, 5,814,648, 5,780,026, 5,776,946, 5,760,002, 5,750,543, 5,741,798, 5,739,279, 5,733,939, 5,723,481, 5,716,967, 5,688,949, 5,686,488, 5,686,471, 5,686,434, 5,684,204, 5,684,041, 5,684,031, 5,684,002, 5,677,318, 5,674,891, 5,672,620, 5,665,752, 5,656,661, 5,635,516, 5,631,283, 5,622,948, 5,618,835, 5,607,959, 5,593,980, 5,593,960, 5,580,888, 5,552,424, 5,552,422, 5,516,764, 5,510,361, 5,508,026, 5,500,417, 5,498,405, 5,494,927, 5,476,876, 5,472,973, 5,470,885, 5,470,842, 5,464,856, 5,464,849, 5,462,952, 5,459,151, 5,451,686, 5,444,043, 5,436,265, 5,432,181, 5,393,756, 5,380,738, 5,376,670, 5,360,811, 5,354,768, 5,348,957, 5,347,029, 5,340,815, 5,338,753, 5,324,648, 5,319,099, 5,318,971, 5,312,821, 5,302,597, 5,298,633, 5,298,522, 5,298,498, 5,290,800, 5,290,788, 5,284,949, 5,280,045, 5,270,319, 5,266,562, 5,256,680, 5,250,700, 5,250,552, 5,248,682, 5,244,917, 5,240,929, 5,234,939, 5,234,937, 5,232,939, 5,225,571, 5,225,418, 5,220,025, 5,212,189, 5,212,172, 5,208,250, 5,204,365, 5,202,350, 5,196,431, 5,191,084, 5,187,175, 5,185,326, 5,183,906, 5,177,079, 5,171,864, 5,169,963, 5,155,122, 5,143,929, 5,143,928, 5,143,927, 5,124,455, 5,124,347, 5,114,958, 5,112,846, 5,104,656, 5,098,613, 5,095,037, 5,095,019, 5,086,064, 5,081,261, 5,081,147, 5,081,126, 5,075,330, 5,066,668, 5,059,602, 5,043,457, 5,037,835, 5,037,811, 5,036,088, 5,013,850, 5,013,751, 5,013,736, 5,006,542, 4,992,448, 4,992,447, 4,988,733, 4,988,728, 4,981,865, 4,962,119, 4,959,378, 4,954,519, 4,945,099, 4,942,236, 4,931,457, 4,927,835, 4,912,248, 4,910,192, 4,904,786, 4,904,685, 4,904,674, 4,904,671, 4,897,397, 4,895,953, 4,891,370, 4,870,210, 4,859,686, 4,857,644, 4,853,392, 4,851,412, 4,847,303, 4,847,290, 4,845,242, 4,835,166, 4,826,990, 4,803,216, 4,801,598, 4,791,129, 4,788,205, 4,778,818, 4,775,679, 4,772,703, 4,767,776, 4,764,525, 4,760,051, 4,748,153, 4,725,616, 4,721,712, 4,713,393, 4,708,966, 4,695,571, 4,686,235, 4,686,224, 4,680,298, 4,678,802, 4,652,564, 4,644,005, 4,632,923, 4,629,793, 4,614,741, 4,599,360, 4,596,828, 4,595,694, 4,595,686, 4,594,357, 4,585,755, 4,579,866, 4,578,390, 4,569,942, 4,567,201, 4,563,476, 4,559,348, 4,558,067, 4,556,672, 4,556,669, 4,539,326, 4,537,903, 4,536,503, 4,518,608, 4,514,415, 4,512,990, 4,501,755, 4,495,197, 4,493,839, 4,465,687, 4,440,779, 4,440,763, 4,435,420, 4,412,995, 4,400,534, 4,355,034, 4,335,141, 4,322,420, 4,275,064, 4,244,963, 4,235,908, 4,234,593, 4,226,887, 4,201,778, 4,181,720, 4,173,650, 4,173,634, 4,145,444, 4,128,664, 4,125,612, 4,124,726, 4,124,707, 4,117,135, 4,027,031, 4,024,284, 4,021,553, 4,021,550, 4,018,923, 4,012,527, 4,011,326, 3,998,970, 3,998,954, 3,993,763, 3,991,212, 3,984,405, 3,978,227, 3,978,219, 3,978,202, 3,975,543, 3,968,224, 3,959,368, 3,949,082, 3,949,081, 3,947,475, 3,936,450, 3,934,018, 3,930,005, 3,857,955, 3,856,962, 3,821,377, 3,821,401, 3,789,121, 3,789,123, 3,726,978, 3,694,471, 3,691,214, 3,678,169, 3,624,216;
Immunosuppressive agents: As disclosed in U. S. Pat. Nos: 4,450,159, 4,450,159, 5,905,085, 5,883,119, 5,880,280, 5,877,184, 5,874,594, 5,843,452, 5,817,672, 5,817,661, 5,817,660, 5,801,193, 5,776,974, 5,763,478, 5,739,169, 5,723,466, 5,719,176, 5,696,156, 5,695,753, 5,693,648, 5,693,645, 5,691,346, 5,686,469, 5,686,424, 5,679,705, 5,679,640, 5,670,504, 5,665,774, 5,665,772, 5,648,376, 5,639,455, 5,633,277, 5,624,930, 5,622,970, 5,605,903, 5,604,229, 5,574,041, 5,565,560, 5,550,233, 5,545,734, 5,540,931, 5,532,248, 5,527,820, 5,516,797, 5,514,688, 5,512,687, 5,506,233, 5,506,228, 5,494,895, 5,484,788, 5,470,857, 5,464,615, 5,432,183, 5,431,896, 5,385,918, 5,349,061, 5,344,925, 5,330,993, 5,308,837, 5,290,783, 5,290,772, 5,284,877, 5,284,840, 5,273,979, 5,262,533, 5,260,300, 5,252,732, 5,250,678, 5,247,076, 5,244,896, 5,238,689, 5,219,884, 5,208,241, 5,208,228, 5,202,332, 5,192,773, 5,189,042, 5,169,851, 5,162,334, 5,151,413, 5,149,701, 5,147,877, 5,143,918, 5,138,051, 5,093,338, 5,091,389, 5,068,323, 5,068,247, 5,064,835, 5,061,728, 5,055,290, 4,981,792, 4,810,692, 4,410,696, 4,346,096, 4,342,769, 4,317,825, 4,256,766, 4,180,588, 4,000,275, 3,759,921;
Iminosugars: deoxynojirimycin or a deoxynojirimycin derivative, like N-propyldeoxynojirimycin, N-butyldeoxynojirimycin, N-butyldeoxygalactonojirimycin, N-pentlydeoxynojirimycin, N-heptyldeoxynojirimycin, N-pentanoyldeoxynojirimycin, N-(5-adamantane-1-ylmethoxy)pentyl)-deoxynojirimycin, N-(5-cholesteroxypentyl)-deoxynojirimycin, N-(4-adamantanemethanylcarboxy-1-oxo)-deoxynojirimycin, N-(4-adamantanylcarboxy-1-oxo)-deoxynojirimycin, N-(4-phenantrylcarboxy-1-oxo)-deoxynojirimycin, N-(4-cholesterylcarboxy-1-oxo)-deoxynojirimycin, or N-(4-β-cholestanylcarboxy-1-oxo)-deoxynojirimycin;
Ceramide analogs: D-threo-1-phenyl-2-palmitoylamino-3-pyrrolidino-1-propanol (P4) or a P4 derivative, like D-threo-4'-hydroxy-1-phenyl-2-palmitoylamino-3-pyrrolidino -1-propanol (4'-hydroxy-P4), D-threo-1-(3,4-trimethylenedioxy)phenyl-2-palmitoylami no-3-pyrrolidino-1-propanol (trimethylenedioxy-P4), D-threo-1-(3',4'-methylenedioxy)phenyl-2-palmitoylamino- 3-pyrrolidino-1-propanol (methylenedioxy-P4) and D-threo-1-(3,4-ethylenedioxy)phenyl-2-palmitoylamino-3 -pyrrolidino-1-propanol (ethylenedioxy-P4 or D-t-et-P4);
In an alternative embodiment of the conjugates of the disclosure, the agent is an agent comprising a peptide or a polypeptide (protein), such as growth factors, cytokines, enzymes, antibodies, antibody fragments, and the like. Specific (poly)peptide drugs and compounds of interest from which the drug moiety may be derived include, but are not limited to:
   Blood and hemopoietic system drugs: antianemia drugs, blood coagulation drugs (hemostatics, anticoagulants, antithrombotics, thrombolytics, blood proteins and their fractions), hemoglobin;
   Cytokines: Intron® or alpha-interferon; Proleukin® IL-2 or aldesleukin, interferon-alpha, interferon-beta (Avonex® or interferon beta-la; Betaseron® / Betaferon® or interferon beta-lb; Rebif® or interferon-beta-la), interferon-gamma, interleukin 1 (IL-1), interleukin 2 (IL-2), interleukin 3 (IL-3), interleukin 4 (IL-4), interleukin 5 (IL-5), interleukin 6 (IL-6), TNF, granulocyte macrophage colony stimulating factor (GM-CSF: Leukine® or sargramostim), granulocyte colony stimulating factor (G-CSF: Neupogen® or filgrastim), macrophage colony stimulating factor (M-CSF), platelet-derived growth factor (PDGF);
   Enzymes: Cerezyme® or glucocerebrosidase; Aldurazyme™ or laronidase; Aryplase™ or arylsulfatase B; I2S or iduronate-2-sulfatase; alpha-L-iduronidase; N-acetylgalactosamine 4-sulfatase; phenylase; aspartylglucosaminidase; acid lipase; cysteine transporter; Lamp-2; alpha galactosidase A; acid ceramidase; alpha-L-fucosidase; ss-hexosaminidase A; GM2-activator deficiency; alpha-D-mannosidase; ss-D-mannosidase; arylsulphatase A; saposin B; neuraminidase; alpha-N-acetylglucosaminidase phosphotransferase; phosphotransferase 7-subunit; heparan-N-sulphatase; a-N-acetylglucosaminidase; acetylCoA: N- acetyltransferase; N-acetylglucosamine 6-sulphatase; galactose 6-sulphatase; 0-galactosidase; hyaluronoglucosaminidase; multiple sulphatases; palmitoyl protein thioesterase; tripeptidyl peptidase I; acid sphingomyelinase; cholesterol trafficking; cathepsin K; alpha-galactosidase B; sialic acid transporter; SOD or Cu/Zn superoxide dismutase; Neprilysin;
   (Organophosphate) detoxifying or scavenging agents: rhodanese, paraoxonase; posphotriesterase; butyrylcholinesterase; organophosphorus acid anhydrolase; or non-polypeptide scavenger like pentetic acid or diethylene triamine pentaacetic acid (DTPA); oximes;
   Brain-acting hormones and neurotransmitters: somatostatin, oxytocin, vasopressin, guaranine, VIP, adrenocorticotropic hormone (ACTH), cholecystokinin (CCK), substance-P, bombesin, motilin, glicentin, glucagon, glucagon-like peptide (GLP-1), leptin;
   Neuropeptides and derivatives thereof: peptide YY (PYY), neuropeptide Y (NPY), pancreatic polypeptide (PP), neurokinin A, neurokinin B, endorphin, enkephalin, met-enkephalin (Tyr-Gly-Gly-Phe-Met), dalargin, loperamide, endomorphin-1 and 2, neurotensin, neuromedin K, neuromedin L, calcitonin related peptide (CGRP), endothelin, ANP ("actrial natriuretic peptide"), BNP ("brain natriuretic peptide"), CNP (C-type natriuretic peptide"), and PACAP ("pituitary adenylate cyclase activating peptide"), TAPP (H-Tyr-D-Ala-Phe-Phe-NH2), senktide (sequence DFFGLM with modifications: Asp-1 = Succinyl-Asp, Phe-3 = Me-Phe, Met-6 = C-terminal amide);
   Neurotrophic factors: NGF or nerve growth factor; BDNF or brain-derived neurotrophic factor; NT3 or neurotrophin-3; NT4 or neurotrophin-4; NT5 or neurotrophin-5; RDGF or retina-derived growth factor; CNTF or ciliary neurotrophic factor; activin; bFGF or basic fibroblast growth factor; aFGF or acidic fibroblast growth factor; GDNF or glial cell line-derived neurotrophic factor or neublastin or artemin or enovin, presephin, neurturin; CTGF or connective tissue growth factor; EGF or epithelial growth factor); erythropoietins (EPO) (Procrit® / Eprex® or erythropoietin alfa; Epogen® or erythropoietin; NeoRecormon® or erythropoietin beta; Aranesp® or darbepoietin alfa); growth hormone or somatotropin (Humatrope®; Protropin® / Nutropin®; Serostim®; Saizen®); anti-NogoA Mab (IN-1); Nogo-A, B or C antagonist, or Nogo66 inhibitor (NEP1-40); Lingo-1; FGL peptide (pGlu-Val-Tyr-Val-Val-Ala-Glu-Asn-Gln-Gln-Gly-Lys-Ser-Lys-Ala, or EVYVVAENQQGKSKA); NAP (Asn-Ala-Pro-Val-Ser-Ile-Pro-Gln, single amino acid letter code, NAPVSIPQ); ADNF-9 (Ser-Ala-Leu-Leu-Arg-Ser-Ile-Pro-Ala, SALLRSIPA);
   Antibodies: 3F8, Abagovomab, Abatacept (Orencia), Abciximab (ReoPro), ACZ885 (canakinumab), Adalimumab (Humira), Adecatumumab, Aflibercept, Afutuzumab, Alacizumab pegol, Alemtuzumab (Campath), Altumomab, Afelimomab, Anatumomab mafenatox, Anrukinzumab (IMA-638), Apolizumab, Arcitumomab, Aselizumab, Atlizumab, Atorolimumab, Bapineuzumab, Basiliximab (Simulect), Bavituximab, Bectumomab (LymphoScan), Belatacept, Belimumab (LymphoStat-B), Bertilimumab, Besilesomab, Bevacizumab (Avastin), Biciromab brallobarbital, Bivatuzumab mertansine, Blinatumomab, Canakinumab, Cantuzumab mertansine, Capromab pendetide (Prostascint), Catumaxomab (Removab), Cedelizumab, Certolizumab pegol (Cimzia), Cetuximab (Erbitux), Citatuzumab bogatox, Cixutumumab, Claretuzumab tetraxetan, Clenoliximab, Clivatuzumab tetraxetan, CNTO 148 (golimumab), CNTO 1275 (ustekinumab), Conatumumab, Dacetuzumab, Dacliximab or Daclizumab (Zenapax), Denosumab, Detumomab, Dorlimomab aritox, Dorlixizumab, Drimakinzumab, Ecromeximab, Eculizumab (Soliris), Edobacomab, Edrecolomab (Panorex), Efalizumab (Raptiva), Efungumab (Mycograb), Elsilimomab, Enlimomab pegol, Epitumomab cituxetan, Epratuzumab, Erlizumab, Ertumaxomab (Rexomun), Etanercept (Enbrel), Etaracizumab, Exbivirumab, Fanolesomab (NeutroSpec), Faralimomab, Felvizumab, Figitumumab, Fontolizumab (HuZAF), Foravirumab, Galiximab, Gantenerumab, Gavilimomab, Gemtuzumab ozogamicin (Mylotarg), Ginakinzumab, Golimumab, Gomiliximab, Ibalizumab, Ibritumomab tiuxetan (Zevalin), Igovomab, Imciromab, Infliximab (Remicade), Inolimomab, Inotuzumab ozogamicin, Ipilimumab, Iratumumab, Ixutumumab, Keliximab, Labetuzumab, Lemalesomab, Lebrilizumab, Lerdelimumab, Lexatumumab, Libivirumab, Lintuzumab, Lucatumumab, Lumiliximab, Mapatumumab, Maslimomab, Matuzumab, Mepolizumab (Bosatria), Metelimumab, Milatuzumab, Minretumomab, Mitumomab, Morolimumab, Motavizumab (Numax), Muromonab, MYO-029 (stamulumab), Nacolomab tafenatox, Naptumomab estafenatox, Natalizumab (Tysabri), Nebacumab, Nerelimomab, Nimotuzumab (BIOMAbEGFR), Nofetumomab merpentan (Verluma), Ocrelizumab, Odulimomab, Ofatumumab, Omalizumab (Xolair), Oregovomab (OvaRex), Otelixizumab, Pagibaximab, Palivizumab (Synagis), Pamtuzumab tetraxetan, Panitumumab (Vectibix), Pascolizumab, Pemtumomab (Theragyn), Pertuzumab (Omnitarg), Pexelizumab, Pintumomab, Priliximab, Pritumumab, PRO 140, Rafivirumab, Ramucirumab, Ranibizumab (Lucentis), Raxibacumab, Regavirumab, Reslizumab, Rilonacept (Arcalyst), Rintalizumab, Rituximab (MabThera, Rituxan), Robatumumab, Rovelizumab, Ruplizumab, Satumomab, Sevirumab, Sibrotuzumab, Siplizumab, Sonepcizumab, Sontuzumab, Stamulumab, Stolanezumab, Sulesomab (LeukoScan), Tacatuzumab tetraxetan, Tadocizumab, Talizumab, Tanezumab, Taplitumomab paptox, Tefibazumab (Aurexis), Telimomab aritox, Tenatumomab, Teneliximab, Teplizumab, TGN1412, Ticilimumab (tremelimumab), Tigatuzumab, TNX-355 (ibalizumab), TNX-650, TNX-901 (talizumab), Tocilizumab (Actemra), Toralizumab, Tositumomab (Bexxar), Trastuzumab (Herceptin), Tremelimumab, Tucotuzumab celmoleukin, Tuvirumab, Urtoxazumab, Ustekinumab, Vapaliximab, Vedolizumab, Veltuzumab, Vepalimomab, Vimakinzumab, Visilizumab (Nuvion), Volociximab, Vontakinzumab, Votumumab (HumaSPECT), Zalutumumab, Zanolimumab (HuMax-CD4), Ziralimumab, Zolimomab aritox, or the functional (epitope-binding) fragments thereof;
In an alternative embodiment of the conjugates of the disclosure, the agent is an agent comprising an oligo- or poly-nucleotide. An agent comprising an oligo- or polynucleotide may be any one of a DNA vaccine, an antisense oligonucleotide, a ribozyme, a catalytic DNA (DNAzyme) or RNA molecule, an siRNA or an expression construct encoding therefor. A DNA vaccine is herein understood to mean an nucleic acid construct comprising a sequence encoding a specific antigen, that is capable of expressing the antigen upon introduction of the construct into a cell of a host organism that is to be vaccinated with the DNA vaccine.

### Ligands

The second entity in the conjugates of the invention is a ligand for a glutathione transporter. The ligand is selected from the group consisting of: glutathione, S-(p-bromobenzyl)glutathione, gamma-(L-gamma-azaglutamyl)-S-(p-bromobenzyl)-L-cysteinylglycin, S-butylglutathione, S-decylglutathione, glutathione reduced ethyl ester, glutathionesulfonic acid, S-hexylglutathione, S-lactoylglutathione, S-methylglutathione, S-(4-nitrobenzyl)glutathione, S-octylglutathione, S-propylglutathione, n-butanoyl gamma-glutamylcysteinylglycine, ethanoyl gamma-glutamylcysteinylglycine, hexanoyl gamma-glutamylcysteinylglycine, octanoyl gamma-glutamylcysteinylglycine, dodecanoyl gamma-glutamylcysteinylglycine, GSH monoisopropyl ester (N-(N-L-glutamyl-L-cysteinyl)glycine 1-isopropyl ester sulfate monohydrate) and glutathione derivatives of the formula I: 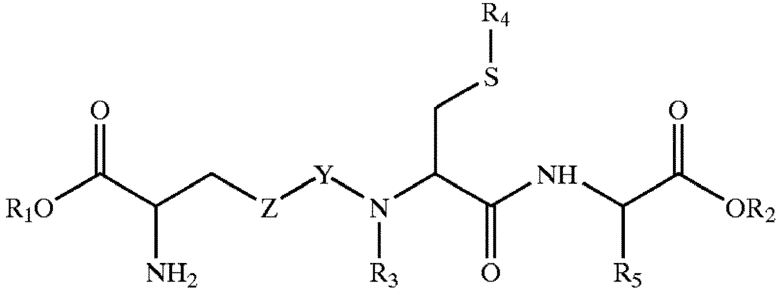
wherein Z=CH₂ and Y=CH₂, or Z=O and Y = C=O;
R₁ and R₂ are independently selected from the group consisting of:
   i) H,
   ii) linear or branched alkyl (1-25C),
   iii) aralkyl (6-26C),
   iv) cycloalkyl (6-25C),
   v) heterocycles (6-20C),
   vi) ethers or polyethers (3-25C), and
   vii) where R₁-R₂ together have 2-20C atoms and form a macrocycle with the remainder of formula I;
R₃ is selected from the group consisting of H and CH₃;
R4 is selected form the group consisting of 6-8C alkyl, benzyl, naphthyl and a therapeutically active compound; and,
   R5 is selected from the group consisting of H, phenyl, CH₃- and CH₂-phenyl; or a pharmaceutically acceptable salt thereof. Preferably the glutathione transporter mediates at least one of specific binding, endocytosis and transcytosis of the ligand and the conjugate comprising the ligand into and/or through a target cell expressing the transporter. Transporter- or receptor-mediated delivery is one possible targeted drug delivery technique that was developed in recent years. It has the potential advantage of high specificity of delivery to target cells which express a receptor/transporter for the ligand that is conjugated with a drug or a drug carrier. The specific targeting of low molecular weight, as well as polypeptide and nucleic-acid based therapeutic or diagnostic agents, and nanocontainers comprising these agents, to cells and tissues may be enhanced greatly through the use of transporter/receptor-mediated delivery.

In one embodiment the ligand in the conjugates of the invention is a ligand for a glutathione transporter that is expressed on endothelial cells of a blood-tissue barrier, including e.g. the blood-testes barrier and blood-CNS barriers, such as e.g. the blood-brain barrier, the blood-cerebral spinal fluid (CSF) barrier, the pial vessel-CSF barrier, the ependyma and glia limitans, the blood-retina barrier, the blood-nerve barrier, and the blood-spinal cord barrier. A preferred ligand is a ligand for a glutathione transporter that is expressed on endothelial cells of the blood-brain barrier and/or brain parenchymal cells (neurons and neuroglia). Use of such ligands will allow the specific delivery, or specifically enhanced delivery, of such targeted agents to the central nervous system (CNS) for the treatment of brain diseases. Receptor-mediated targeting may further be combined with non-specific drug delivery systems (like protein conjugates, PEGylation, nanoparticles, liposomes, and the like) to greatly improve the pharmacokinetic and biodistribution properties of the drugs, which will significantly redirect the drugs specifically to receptor-expressing cells, tissues and organs, including the ones protected by specific blood-tissue barriers like e.g., the CNS, the blood-brain barrier (BBB), the retina and the testes.

In a preferred embodiment therefore, the ligand that is to be incorporated in the conjugates of the invention, is a ligand for an endogenous glutathione transporter on a target cell. The ligand preferably is a ligand for a glutathione transporter of a vertebrate target cell, more preferably a glutathione transporter of a mammalian target cell, and most preferably a glutathione transporter of a human target cell. The ligand preferably is a ligand that specifically binds to the glutathione transporter. More preferably, the ligand specifically binds to the Na-dependent GSH transporter as present in human cerebrovascular endothelial cells as described by Kannan et al. (2000, Brain Res. 852(2):374-82). Specific binding of a ligand to a transporter preferably is as defined herein above. In another embodiment the ligand is a ligand that is endocytosed and/or transcytosed into and/or through the target cell as may be assayed by a cell culture model of the BBB (using primary isolated bovine brain capillary endothelial cells (BCEC)) as described by Gaillard et al. (2001, Eur J Pharm Sci. 12(3): 215-222), or similar models using e.g., RBE4 cells, or MDCK cells as target cells. A ligand that is endocytosed and/or transcytosed into and/or through the target cell is herein defined as a ligand that is endocytosed or transcytosed into or through a BCEC or MDCK target cell at a rate that is at least 5, 10, 20 or 50% enhanced as compared to control conditions selected from a) cells lacking expression of GSH transporters; b) cells pretreated with excess of free GSH; and c) a reference compound lacking a GSH moiety; when measured at 15, 30, or 60 minutes or 1, 2, 4, 8, or 18 hours or less after addition of the ligand to the target cell. Alternatively, endocytosis and/or transcytosis of GSH transporter-targeted ligands may be assayed by in vivo bioimaging techniques using for instance near-infrared dyes or radioactive labels conjugated thereto, resulting in at least 10, 20, or 50% enhanced retention in CNS area of the ligand at given time-points (based on region of interest (ROI) pixel quantification), as compared to appropriate control conditions (e.g., comparison to reference compounds lacking GSH moieties).

Preferred ligands that bind to the glutathione transporter, for use in accordance with the present invention include e.g. ligands selected from the group consisting of: glutathione (GSH or gamma-glutamylcysteinylglycine), S-(p-bromobenzyl)glutathione, gamma-(L-gamma-azaglutamyl)-S-(p-bromobenzyl)-L-cysteinylglycin, S-Butylglutathione, S-Decylglutathione, Glutathione reduced ethyl ester, Glutathionesulfonic acid, S-Hexylglutathione, S-Lactoylglutathione, S-Methylglutathione, S-(4-Nitrobenzyl)glutathione, S-Octylglutathione, S-Propylglutathione, n-butanoyl gamma-glutamylcysteinylglycine (also known by the abbreviation GSH-C4) or the ethanoyl, hexanoyl, octanoyl or dodecanoyl derivatives thereof (also known by the abbreviations GSH-C2, GSH-C6, GSH-C8 and GSH-C12, respectively), GSH monoisopropyl ester (also known as N-(N-L-glutamyl-L-cysteinyl)glycine 1-isopropyl ester sulfate monohydrate or YM737), and GSH derivatives as described in U.S. Pat No 6,747,009 of the formula I: 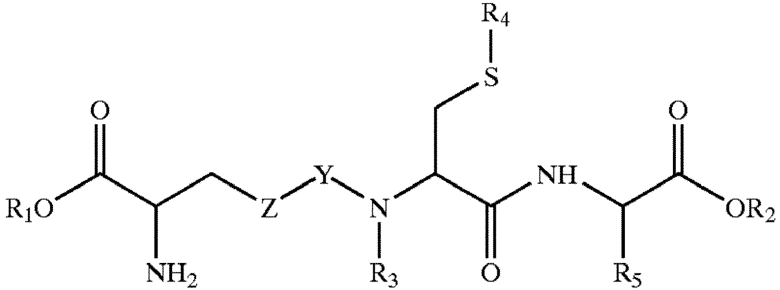
wherein Z=CH₂ and Y=CH₂, or Z=O and Y=C,
R₁ and R₂ are independently selected from the group consisting of H, linear or branched alkyl (1-25C), aralkyl (6-26C), cycloalkyl (6-25C), heterocycles (6-20C), ethers or polyethers (3-25C), and where R₁-R₂ together have 2-20C atoms and form a macrocycle with the remainder of formula I;
R₃ is selected from the group consisting of H and CH₃,
R4 is selected form the group consisting of 6-8C alkyl, benzyl, naphthyl and a therapeutically active compound, and
R5 is selected from the group consisting of H, phenyl, CH₃ and CH2phenyl or a pharmaceutically acceptable salt thereof.

In a preferred embodiment R₃ in the formula above is H. In a further preferred embodiment R₄ in the formula above is benzyl. In yet a further preferred embodiment R₅ in the formula above is phenyl.

In one preferred embodiment of the invention, the ligand is conjugated or synthesized via the N-terminal amino acid residue, i.e. the amine group of the glutamic acid residue.

In another preferred embodiment of the invention, the ligand is conjugated or synthesized via the C-terminal amino acid residue, i.e. the carboxyl group of the glycine residue.

In yet another preferred embodiment of the invention, the ligand is conjugated or synthesized via the thiol (SH) group of the cysteine moiety.

### Nanocontainers

The ligands in the conjugates of the disclosure may be conjugated directly to the agents, or alternatively, the ligands may be conjugated to pharmaceutically acceptable nanocontainers that comprises the agents. In such conjugates, the agents may e.g. be encapsulated within nanocontainers, such as nanoparticles, liposomes or nanogels, whereby the ligand is preferably conjugated coupled to such a nanocontainer. The ligands in the conjugates of the invention are conjugated to pharmaceutically acceptable nanocontainers that comprises the agents. In such conjugates, the agent is encapsulated within nanocontainers, being liposomes, whereby the ligand is conjugated to such a nanocontainer. Such conjugation to the nanocontainer may be either directly or via any of the well-known polymeric conjugation agents such as sphingomyelin, polyethylene glycol (PEG) or other organic polymers. Details of producing such pharmaceutical compositions comprising targeted (PEG) liposomes are described in US Patent No.6,372,250. Thus, in a preferred embodiment a conjugate according to disclosure is a conjugate wherein the pharmaceutically acceptable carrier comprises at least one of: a carrier protein, a nanocontainer, a liposome, a polyplex system, a lipoplex system, and, polyethyleneglycol.

In conjugates of the disclosure wherein the agent comprises a poly- or oligonucleotide, the pharmaceutically acceptable carrier preferably is a lipoplex system comprising at least one of cationic lipids or amphoteric lipids (as detailed in WO2002/066012), or a polyplex system comprising at least one of poly-L-Lysine, poly-L-ornithine, polyethyleneimine, and polyamidoamine. There are two major kinds of non-viral delivery systems for the intracellular delivery of nucleic acid based antiviral drugs (like DNA vaccines, antisense oligonucleotides, ribozymes, catalytic DNA (DNAzymes) or RNA molecules, siRNAs or plasmids encoding thereof), comprising lipoplex systems (cationic liposomes containing DNA) and polyplex systems (DNA attached to a cationic polymer). In a preferred embodiment of the disclosure, the pharmaceutical acceptable carrier is a lipoplex system or a polyplex system. In addition, the pharmaceutical acceptable carrier may further preferably comprise a protein conjugate, polyethyleneglycol (PEGylation), a nanoparticle or a liposome. Polyplex systems comprise cationic polymers such as poly-L-Lysine (PLL), poly-L-ornithine (POL), polyethyleneimine (PEI), polyamidoamine (PAM) or combinations thereof with DNA. Polycationic systems enter cells mainly by adsorptive or fluid-phase endocytosis. Cationic polymers, including PEI, have the ability to condens DNA and to destabilize the membrane potential. Moreover, it has been shown that plasmid delivery by PEI polyplex systems could be achieved by controlling the physical chemical and biological properties of the complex. However, transfection efficiency and gene expression are limited compared to viral transduction systems. Since PEI systems may perturb membranes they can cause also toxicity that correlates with the molecular weight and the nuclear concentration of the polymer. In this respect it was shown that linear PEI (22 kDa) was more toxic than branched PEI (25 kDa) and also related to the amount of PEI used in polyplex systems as expressed by the N/P ration (amount of nitrogen in the polymer related to the amount of DNA). Others state that linear PEI polyplex systems exhibited improved cell viability and higher transfection efficiency. Recently various biodegradable PEI-derivatives have been synthesized with better transfection properties and less toxicity than linear PEI. Overall, the efficacy of PEI and probably of polycationic systems in general depends on the molecular weight, the overall cationic charge and the degree of branching. When attached to DNA, other factors like the amount of DNA, the particle size and the zeta potential are important features. Furthermore, the positively charged polycationic systems interact readily with the negatively charged plasma proteins when administered intravenously and opsonization occurs following binding to blood proteins which target them to be cleared by the reticulo-endothelial system (RES). Particulary, the formation of aggregates leads to the uptake by phagocytic cells and the entrapment by capillary networks (mainly lungs following intravenous administration) that results in a fast clearance from the plasma compartment and a poor transfection of target tissues/organs. However, PEGylation can dramatically reduce this. Furthermore, application of a targeting/internalization ligand avoids the need to apply polyplex systems with a large N/P ratio and therefore a high overall positive charge and may therefore reduce many problems that are associated with cationic polymers (such as toxicity, binding to blood constituents). Naked lipoplex systems are also readily opsonized by serum components and cleared by similar mechanisms as polyplex systems e.g. by the reticulo-endothelial system (RES). Moreover, although the unmethylated CpGs of lipoplex systems are masked preventing an innate immune response, once they are in the general circulation, lipoplex systems may be, similarly like polyplex systems, opsonized by blood proteins (C3, IgG, lipoproteins and fibronectin) resulting in inflammatory reactions (mediated by TNF-alpha, IL-6 and IL-12) in lungs and liver. In addition, complex activation and activation of T-, B-, NK-cells and macrophages has been found and were related to the injected dose of the lipoplex. Next to reducing the number of unmethylated CpGs, such interactions can be limited by PEGylation of these systems or by using immunosuppressive agents (e.g. dexamethasone). In addition, the kinetics of these systems were considerably improved by PEGylation reducing their systemic clearance and increasing targeting efficiency (by application of selective/specific targeting ligands). Moreover, decreasing the size of lipoplex systems seems to be a key factor in their tissue distribution and cellular uptake and increases their transfection efficiency. Generally, the tissue distribution and the persistence of expression of lipoplex and polyplex systems is mainly dependent, like with small molecular drugs following parenteral administration, also on the pharmacokinetics (clearance, distribution volume), the formulation (size, charge, PEGylation, etc.) and the dosage regimen (high volume bolus, sequential injection, constant infusion). With respect to dosage regimen it is interesting to note that sequential injection of lipoplexes and plasmid DNA resulted in higher expression but also minimized cytokine induction. Furthermore, the delivery to the target tissue/organ is depending on blood flow and tissue/organ uptake or permeability and the balance of clearances of target and non-target tissues/organs. Therefore a proper dosage regimen, based on pharmacokinetic parameters, should be designed to optimize delivery/targeting to organs and tissues. In addition, this should be harmonized with respect to the intracellular pharmacokinetics. The intracellular pharmacokinetics (distribution, elimination) of lipoplex and polyplex systems following cellular uptake is an important issue. Apart from receptor-mediated uptake, the internalization (via the clathrin- or the caveolae-dependent route) of particularly untargeted PEI-systems seem to depend on both cell line and the PEI-polyplex type (linear PEI vs branched PEI). Frequently, such systems end up in late endosomes therefore they need to escape from these organelles to enter the cytoplasm to ultimately reach the nucleus. Cationic systems like polyplex systems can escape from the endosomes/lysosomes because they have the ability to buffer pH and cause osmotic swelling of these organelles according to the so-called "proton sponge-mediated escape" theory. Nevertheless, it seems that a small fraction of the internalized systems escapes into the cytoplasm and that a large part stays in the endosomes/lysosomes and is degraded. However, it has been shown that incorporation of fusogenic lipids or cationic peptides (mellitin) into these systems could enhance their endosomal escape. Once in the cytosol linear plasmids can be readily degraded by nucleases while circular plasmids are much more stable. Circular (desoxy)nucleic acid molecules are therefore preferred. Particularly calcium sensitive nucleases seem to be responsible for this degradation. Ultimately the plasmids have to be transported into the nucleus via the nuclear pore complex (NPC) which forms an aqueous channel through the nuclear envelope and it was estimated that about 0.1% of plasmids are able to enter the nucleus from the cytosol. Molecules smaller than 40 kDa can passively pass the NPC while larger molecules (> 60 kDa) need a specific nuclear localization signal (NLS) to be actively transported through the NPC permitting transport of molecules up to 25-50 MDa. Indeed it was shown that coupling of an NLS to plasmids enhanced the nuclear accumulation and expression of plasmid DNA. Preferably therefore, an NLS is coupled to any expression construct for use in the conjugates of the disclosure.

A large variety of methods for conjugation of ligands with the agents or carriers are known in the art. Such methods are e.g. described by Hermanson (1996, Bioconjugate Techniques, Academic Press), in U.S. 6,180,084 and U.S. 6,264,914 and include e.g. methods used to link haptens to carrier proteins as routinely used in applied immunology (see Harlow and Lane, 1988, "Antibodies: A laboratory manual", Cold Spring Harbor Laboratory Press, Cold Spring Harbor, NY). It is recognised that, in some cases, a ligand or agent may lose efficacy or functionality upon conjugation depending, e.g., on the conjugation procedure or the chemical group utilised therein. However, given the large variety of methods for conjugation the skilled person is able to find a conjugation method that does not or least affects the efficacy or functionality of the entities to be conjugated. Suitable methods for conjugation of a ligand with an agent or carrier include e.g. carbodiimide conjugation (Bauminger and Wilchek, 1980, Meth. Enzymol. 70: 151-159). Alternatively, an agent or carrier can be coupled to a ligand as described by Nagy et al., Proc. Natl. Acad. Sci. USA 93:7269-7273 (1996); and Nagy et al., Proc. Natl. Acad. Sci. USA 95:1794-1799 (1998). Other methods for conjugating that may suitable be used are e.g. sodium periodate oxidation followed by reductive alkylation of appropriate reactants and glutaraldehyde crosslinking. A particularly advantageous method of conjugation may be applied when both the ligand as well as the agent or carrier are (polypeptides. In such instances the two entities may be synthesised as a single (poly)peptide chain comprising the amino acid sequences of both the ligand and the peptide agent or carrier. In addition to covalent bonding, in a conjugate according to the disclosure the agent or carrier may also be directly conjugated to the ligand molecule by non-specific or specific protein-protein interaction, non-covalent bonding and/or coordinating chemical bonding, which conjugation may optionally be effected via a spacer or linker that is bound to the agent and the ligand.

In another aspect, the invention relates to a conjugate of the invention as defined above, for use in the treatment and/or prevention of a CNS disorder. According to the invention, a conjugate of the invention is used in the manufacture of a medicament for the treatment and/or prevention of a CNS disorder. Similarly the disclosure relates to methods for the treatment and/or prevention of a CNS disorder, wherein an effective dose of a conjugate of the invention is administered to a subject in need thereof. The subject in need of treatment or prevention of a CNS disorder may be a vertebrate, mammal, or, preferably a human.

### CNS diseases and related disorders

The following paragraphs provides a description of the various pathologies of the CNS, and associated conditions or related disorders that, in various embodiments of the invention, may be treated and/or prevented with the conjugates of the invention comprising a GSH transporter targeted active agent.

In a preferred embodiment of the invention, the CNS pathology is one of a neurodegenerative disorder, such as Alzheimer's disease (AD), Parkinson's disease (PD), Huntington's disease (HD), multiple sclerosis (MS), amyotrophic lateral sclerosis (ALS), cerebrovascular accidents (CVA: ischemic stroke, intracerebral hemorrhage (ICH) or subarachnoid hemorrhage (SAH)), vascular-related dementia, brain trauma (traumatic brain injury), spinal cord injury, alcoholism, Prion diseases: Creutzfeldt-Jakob disease (CJD), bovine spongiform encephalopathy (BSE).

In one embodiment of the invention, Alzheimer's Disease is treated with one of the following targeted drugs or compounds based on cholinesterase inhibitors: Exelon (rivastigmine), Razadyne ER (galantamine), Debio 9902 SR, NGX267; NMDA Antagonists: Namenda / Axura / Ebixa (memantine), Dimebon (dimebolin), NP-0361; alpha-7 nicotinic acetylcholine agonist: ABT-089, AZD-0328, R-4996 / MEM-63908, EVP-6124; Passive Immunotherapies: Gammagard (IVIG), Bapineuzumab (AAB-001), LY2062430, PF-4360365 (RN1219), AAB-002, ACU-5A5, R-1450, ACI-01-Ab7, BAN-2401; gamma-Secretase Inhibitors / gamma-Secretase Modulators: Flurizan (tarenflurbil; R-flurbiprofen), LY-450139, GSI-953, MK-0752; beta-Secretase Inhibitors: CTS-21166; Anti-TNF: Enbrel (etanercept), Certolizumab pegol (CDP870, tradename Cimzia), Remicade (infliximab) or Humira (adalimumab); Insulin Related: Avandia (rosiglitazone), TTP-488 (RAGE Inhibitor), NGX-96992, Insulin Degrading Enzyme, Ketasyn (AC-1202), SRT-501; Tau / GSK3 Inhibitors: NP031112, Dimebon; Coagulation Cascade: PAI-1 Antagonist; Metal Chelators: PBT2; Statins: Lipitor (atorvastatin), Zocor (simvastatin); Hormonal: Evista (raloxifene); 5-HT / GABA: lecozotan / lecozotan SR, PRX-03140, MK-0249, 742457 (SB-742457); Other Anti-Amyloid: ELND005 (scyllo-inositol; AZD-103), curcumin, caprospinol (SP-233); beta-sheet breaker peptide (Leu-Pro-Phe-Phe-Asp or LPFFP, Ac-LPFFP-NH2 as described in US Patent Nos. 5,948,763 and 6,462,171, or Leu-Pro-Tyr-Phe-Asp-amide or LPYFDa as described in Juhasz et al., 2009, J. Alzheimers Dis. 16: 189-196); or BACE1 inhibitors: anti-BACEl antibodies or fragments thereof: or Neprilysin.

In a preferred embodiment of the invention, the CNS pathology is one of a peripheral disorder with a CNS component, such as septic shock, brain metastasis, hepatic encephalopathy, (diabetic) hypertension, diabetic (micro)angiopathy, sleeping sickness, Whipple disease, Duchenne muscular dystrophy (DMD), aspartylglucosaminuria, cholesterol ester storage disease, Wolman disease, cystinosis, Danon disease, Fabry disease, Farber lipogranulomatosis, Farber disease, fucosidosis, galactosialidosis types I/II, Gaucher disease types I/II/III, Gaucher disease, globoid cell leucodystrophy, Krabbe disease, glycogen storage disease II, Pompe disease, GM1-gangliosidosis types 1/11/11I, GM2-gangliosidosis type I, Tay Sachs disease, GM2-gangliosidosis type II, Sandhoff disease, GM2-gangliosidosis, alpha-mannosidosis types 1/11, mannosidosis, metachromatic leucodystrophy, mucolipidosis type I, sialidosis types 1/11 mucolipidosis types 11/III 1-cell disease, mucolipidosis type IIIC pseudo-Hurler polydystrophy, mucopolysaccharidosis type I, mucopolysaccharidosis type II, Hunter syndrome, mucopolysaccharidosis type IIIA, Sanfilippo syndrome, mucopolysaccharidosis type IIIB, mucopolysaccharidosis type IIIC, mucopolysaccharidosis type IIID, mucopolysaccharidosis type IVA, Morquio syndrome, mucopolysaccharidosis type IVB Morquio syndrome, mucopolysaccharidosis type VI, mucopolysaccharidosis type VII, Sly syndrome, mucopolysaccharidosis type IX, multiple sulphatase deficiency, neuronal ceroid lipofuscinosis, CLN1 Batten disease, Niemann-Pick disease types A/B, Niemann-Pick disease, Niemann-Pick disease type C1, Niemann-Pick disease type C2, pycnodysostosis, Schindler disease types VII, Schindler disease, sialic acid storage disease, and (pre)eclampsia.

In another embodiment of the invention, lysosomal storage diseases (LSDs) are treated with one of the following targeted drugs or compounds based on drugs and compounds that cause reduction of lysosomal stored materials like glucocerebroside, sphingomyelin, ceramide, G M1 -ganglioside, G M2 -ganglioside, globoside, galactosylceramide, dermatan sulfate, heparan sulfate, keratan sulfate, sulfatides, mucopolysaccharides, sialyloligosaccharides, glycoproteins, sialyloligosaccharides, glycolipids, globotriaosylceramide, O-linked glycopeptides, glycogen, free sialic acid, fucoglycolipids, fucosyloligosaccharides, mannosyloligosaccharides, aspartylglucosamine, cholesteryl esters, triglycerides, and ceroid lipofuscin pigments in lysosomal storage diseases, like Gaucher disease (stored glucocerebroside) using targeted enzyme replacement therapy with beta-glucocerebrosidase (e.g., imiglucerase (Cerezyme marketed by Genzyme) or velalglucerase (in development by Shire), gene-activated beta-glucocerebrosidase, or with substrate inhibition of glucosylceramide synthase with imino sugars (e.g., miglustat (Zavesca marketed by Actelion) = N-butyl-deoxynojirimycin (NB-DNJ), N-butyl-galactosyl-deoxynojirimycin (NB-DGJ), N-(5-adamantane-1-yl-methoxypentyl)- deoxynojirimycin (AMP-DNJ), N-(5-adamantane-1-yl-methhoxy-pentyl)deoxynojirimycin (AMP-DNM), or with Glucosylceramide analogs (e.g., Genz 112638: d-threo-ethylendioxyphenyl-2-palmitoylamino-3-pyrrilidino-propanol), or with chaperone therapy (e.g., isofagomine tartrate, AT2101, (to be marketed as Plicera™ by Amicus Therapeutics, chemical name: (3R, 4R, 5R)-3,4-Dihydroxy-5-hydroxymethyl-piperidine); Fabry (stored globotriaosylceramide) using targeted enzyme replacement therapy with alpha-galactosidase A (e.g., Algalsidase alpha (Replagal marketed by Shire), Algalsidase beta (Fabrazyme marketed by Genzyme), or with substrate inhibition of glucosylceramide synthase with imino sugars (e.g., miglustat (Zavesca marketed by Actelion) = N-butyl-deoxynojirimycin (NB-DNJ), N-butyl-galactosyl-deoxynojirimycin (NB-DGJ), N-(5-adamantane-1-yl-methoxypentyl) (AMP-DNJ), AMP-DNM or MZ-21 and MZ-31 from Macrozyme), or with glucosylceramide analogs (e.g., Genz 112638: d-threo-ethylendioxyphenyl-2-palmitoylamino-3-pyrrilidino-propanol), or with chaperone therapy (AT1001, migalastat hydrochloride (to be marketed as Amigal™ by Amicus Therpeutics with chemical names 3,4,5-piperidinetriol, 2-(hydroxymethyl), hydrochloride, (2R,3S,4R,5S)-, (+)-(2R,3S,4R,5S)-2-(hydroxymethyl)piperidine-3,4,5-triol hydrochloride, 1,5-dideoxy-1,5-imino-D-galactitol hydrochloride); MPS I (Hurler-Scheie, stored dermatan sulfate, heparan sulfate) using targeted enzyme replacement therapy with alpha-L- iduronidase (e.g., laronidase (Aldurazyme marketed by Genzyme/Biomarin); MPS II (Hunter, stored dermatan sulfate, heparan sulfate) with targeted enzyme replacement therapy with iduronate-2-sulfatase (e.g., idursulfase (Elaprase marketed by Shire); MPS III A (Sanfilippo A, stored heparan sulfate) using targeted enzyme replacement therapy with heparan sulfamidase; MPS III B (Sanfilippo B, stored heparan sulfate) using targeted enzyme replacement therapy with alpha-N-acetylglucosaminidase; MPS III C (Sanfilippo C, stored heparan sulfate) using targeted enzyme replacement therapy with alpha-glucosaminide N-acetyltransferase; MPS III D (Sanfilippo D, stored heparan sulfate) using targeted enzyme replacement therapy with N-acetylglucosamine-6-sulfate sulfatase; MPS IV (Morquio, stored keratan sulfate) using targeted enzyme replacement therapy with N-acetylgalactosamine-6-sulfatase; MPS VI (Maroteaux-Lamy, stored glycosaminoglycan (GAG)) using targeted enzyme replacement therapy with arylsulfatase B (e.g., galsulfase (Naglazyme marketed by Biomarin); MPS VII (Sly, stored dermatan sulfate, heparan sulfate) using targeted enzyme replacement therapy with beta-glucuronidase; Krabbe (stored galactosylceramide) using targeted enzyme replacement therapy with galactocerebrosidase; Niemann-Pick A and B (stored sphingomyelin) using targeted enzyme replacement therapy with acid sphyngomyelinase (in development by Genzyme); Niemann Pick C (stored sphingomyelin) using targeted substrate inhibition of glucosylceramide synthase with miglustat (Zavesca marketed by Actelion) = N-butyl-deoxynojirimycin (NB-DNJ) or cyclodextrins (especially hydroxypropyl-betacyclodextrin); Metachromatic Leukodystrophy (stored sulfatides) using targeted enzyme replacement therapy with arylsulfatase A; Mucolipidosis II/III (stored Sialyloligosaccharides, glycoproteins, glycolipids) using targeted enzyme replacement therapy with UDP-N-acetylglucosamine or lysosomal enzyme N-acetylglucosamine-1-phosphotransferase (GNPT); GM1 Gangliosidosis (stored GM1 Ganglioside) using targeted enzyme replacement therapy with beta-galactosidase; Sandhoff disease (GM2 Gangliosidosis with stored GM2 Ganglioside) using targeted enzyme replacement therapy with beta-hexosaminidase A, or substrate inhibition of glucosylceramide synthase using imino sugars (e.g., miglustat (Zavesca marketed by Actelion) = N-butyl-deoxynojirimycin (NB-DNJ)); Tay Sachs disease (GM2 Ganglioside) using targeted enzyme replacement therapy with alpha-hexosaminidase A or substrate inhibition of glucosylceramide synthase using imino sugars (e.g., miglustat (Zavesca marketed by Actelion) = N-butyl-deoxynojirimycin (NB-DNJ)).

In yet another preferred embodiment of the invention, the CNS pathology is one of a neuropsychiatric disorders, such as depression (e.g., modified by using brain targeted liposomal mineralocorticoid receptor agonists like fludrocortisone, deoxycorticosterone or aldosterone, thereby reducing peripheral cardiovascular side-effects), autism, anxiety, attention deficit hyperactivity disorder (ADHD), addiction and other substance-related disorders, neuropsychiatric systemic lupus erythematosus, bipolar disorder, eating disorders, schizophrenia, and other psychoses; or another CNS disorders, such as primary brain tumors, epilepsy/seizures, migraine and other headaches (cluster, vascular, tension), narcolepsy, insomnia (and other sleep disorders), chronic fatigue syndrome, mountain sickness, obesitas, bacterial and viral encephalitis, bacterial and viral meningitis, AIDS-related dementia; or an angiogenesis-related disorders, such as vascular tumors, proliferative vitreoretinopathy, rheumatoid arthritis, Crohn's disease, atherosclerosis, ovarian hyperstimulation, psoriasis, endometriosis associated with neovascularisation, restenosis subsequent to balloon angioplasty, scar tissue overproduction, peripheral vascular disease, hypertension, inflammatory vasculitides, Reynaud's disease, Reynaud's phenomenon, aneurysms, arterial restenosis, thrombophlebitis, lymphangitis, lymphedema, wound healing and tissue repair, ischemia reperfusion injury, angina, myocardial infarctions, chronic heart conditions, heart failure such as congestive heart failure, age-related macular degeneration, and osteoporosis.

### Targeting to and/or across blood-tissue barriers

In another aspect of the disclosure, a method of targeted drug delivery of an effective amount of an agent, or a pharmaceutical acceptable carrier comprising an agent, to a target site that is protected by a specific blood-tissue barriers like e.g., the CNS, the blood-brain barrier (BBB), the retina and the testes, is provided wherein: a) the agent or the pharmaceutical acceptable carrier is conjugated to a ligand, that facilitates the specific binding to and internalization by an internalizing GSH uptake receptor of the target site, thereby forming the conjugate as defined above; and b) the agent is delivered at the target site within a time period of about day 1 to about day 5 after administration to a person in need. In a preferred embodiment, the blood-tissue barrier, e.g. blood-brain barrier in the method is not disrupted by administration of agents disrupting the blood-tissue barrier. In another preferred embodiment, the time-period is of about day 1 to about day 7, more preferably of about day 1 to about day 10, even more preferably of about day 1 to about day 14, most preferably of about day 1 to about day 21.

The following paragraphs relate to various embodiments of the invention concerning the active targeting to target sites protected by specific blood-tissue barriers like e.g., the CNS, the blood-brain barrier (BBB), the retina and the testes, by receptor-mediated transcytosis. In a preferred embodiment of the invention, the GSH transporter that mediates at least one of endocytosis and transcytosis is located in (the luminal side of) capillaries in the brain. In general, without wishing to be bound to any theory, receptor-mediated transcytosis occurs in three steps: receptor-mediated endocytosis of the agent at the luminal (blood) side, movement through the endothelial cytoplasm, and exocytosis of the drug at the abluminal (brain) side of the brain capillary endothelium. Upon receptor-ligand internalization, chlathrin-coated vesicles are formed, which are approximately 120 nm in diameter. These vesicles may transport their content to the other side of the cell or go into a route leading to protein degradation. Indeed, at least two important routes for degrading proteins have been identified, including the lysosomal and the ubiquitin-proteasome route. Therefore, to escape from the endosomal□lysosomal system, mechanisms have been applied to ensure release of the drug into the cytosol. These include the application of pH-sensitive liposomes or cationic molecules. Nevertheless, with or without application of lysosomal escape mechanisms, protein delivery to the brain has been shown to be effective. Therefore, receptor-mediated transcytosis allows the specific targeting of larger drug molecules or drug-carrying particles (such as liposomes, polymer systems, nanoparticles) to the brain. In a preferred embodiment, the GSH transporter mediates at least one of endocytosis and transcytosis, the ligand and the pharmaceutical acceptable carrier is selected to bypass lysosomal degradation of the agent in the cell.

### Gene therapy

Some aspects of the disclosure concern the use of expression vectors comprising the nucleotide sequences encoding an agent comprising an oligo- or poly-nucleotide as defined above, wherein the vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; and references cited therein. Particularly suitable gene therapy vectors include Adenoviral and Adeno-associated virus (AAV) vectors. These vectors infect a wide number of dividing and non-dividing cell types. In addition adenoviral vectors are capable of high levels of transgene expression. However, because of the episomal nature of the adenoviral and AAV vectors after cell entry, these viral vectors are most suited for therapeutic applications requiring only transient expression of the transgene (Russell, 2000, J. Gen. Virol. 81: 2573-2604) as indicated above. Preferred adenoviral vectors are modified to reduce the host response as reviewed by Russell (2000, supra).

Generally, gene therapy vectors will be as the expression vectors described above in the sense that they comprise the nucleotide sequence encoding agent to be expressed, whereby the nucleotide sequence is operably linked to the appropriate regulatory sequences as indicated above. Such regulatory sequence will at least comprise a promoter sequence. As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues. Suitable promoters for expression of the nucleotide sequence encoding the polypeptide from gene therapy vectors include e.g. cytomegalovirus (CMV) intermediate early promoter, viral long terminal repeat promoters (LTRs), such as those from murine moloney leukaemia virus (MMLV) rous sarcoma virus, or HTLV-1 , the simian virus 40 (SV 40) early promoter and the herpes simplex virus thymidine kinase promoter.

Several inducible promoter systems have been described that may be induced by the administration of small organic or inorganic compounds. Such inducible promoters include those controlled by heavy metals, such as the metallothionine promoter (Brinster et al. 1982 Nature 296: 39-42; Mayo et al. 1982 Cell 29: 99-108), RU-486 (a progesterone antagonist) (Wang et al. 1994 Proc. Natl. Acad. Sci. USA 91: 8180-8184), steroids (Mader and White, 1993 Proc. Natl. Acad. Sci. USA 90: 5603-5607), tetracycline (Gossen and Bujard 1992 Proc. Natl. Acad. Sci. USA 89: 5547-5551; U.S. Pat. No. 5,464,758; Furth et al. 1994 Proc. Natl. Acad. Sci. USA 91: 9302-9306; Howe et al. 1995 J. Biol. Chem. 270: 14168-14174; Resnitzky et al. 1994 Mol. Cell. Biol. 14: 1669-1679; Shockett et al. 1995 Proc. Natl. Acad. Sci. USA 92: 6522-6526) and the tTAER system that is based on the multi-chimeric transactivator composed of a tetR polypeptide, as activation domain of VP16, and a ligand binding domain of an estrogen receptor (Yee et al., 2002, US 6,432,705).

The gene therapy vector may optionally comprise a second or one or more further nucleotide sequence coding for a second or further protein. The second or further protein may be a (selectable) marker protein that allows for the identification, selection and/or screening for cells containing the expression construct. Suitable marker proteins for this purpose are e.g. fluorescent proteins such as e.g. the green GFP, and the selectable marker genes HSV thymidine kinase (for selection on HAT medium), bacterial hygromycin B phosphotransferase (for selection on hygromycin B), Tn5 aminoglycoside phosphotransferase (for selection on G418), and dihydrofolate reductase (DHFR) (for selection on methotrexate), CD20, the low affinity nerve growth factor gene. Sources for obtaining these marker genes and methods for their use are provided in Sambrook and Russel (2001) "Molecular Cloning: A Laboratory Manual (3rd edition), Cold Spring Harbor Laboratory, Cold Spring Harbor Laboratory Press, New York.

Alternatively, the second or further nucleotide sequence may encode a protein that provides for fail-safe mechanism that allows curing a subject from the transgenic cells, if deemed necessary. Such a nucleotide sequence, often referred to as a suicide gene, encodes a protein that is capable of converting a prodrug into a toxic substance that is capable of killing the transgenic cells in which the protein is expressed. Suitable examples of such suicide genes include e.g. the E.coli cytosine deaminase gene or one of the thymidine kinase genes from Herpes Simplex Virus, Cytomegalovirus and Varicella-Zoster virus, in which case ganciclovir may be used as prodrug to kill the IL-10 transgenic cells in the subject (see e.g. Clair et al., 1987, Antimicrob. Agents Chemother. 31: 844-849). The gene therapy vectors are preferably formulated in a pharmaceutical composition comprising a suitable pharmaceutical carrier as defined below.

### Antibodies

Antibodies or antibody-fragments may be a component part of the conjugates or agents of the disclosure. Antibodies or antibody-fragments may be a component part of the conjugates of the invention. Preferably the antibody or fragment thereof is a monoclonal antibody (MAb). MAbs to complement components can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981). For treating humans, the anti-complement MAbs would preferably be used as chimeric, deimmunised, humanised or human antibodies. Such antibodies can reduce immunogenicity and thus avoid human anti-mouse antibody (HAMA) response. It is preferable that the antibody be IgG4, IgG2, or other genetically mutated IgG or IgM which does not augment antibody-dependent cellular cytotoxicity (S.M. Canfield and S.L. Morrison, J. Exp. Med., 1991: 173: 1483-1491) and complement mediated cytolysis (Y.Xu et al., J. Biol. Chem., 1994: 269: 3468-3474; V.L. Pulito et al., J. Immunol., 1996; 156: 2840-2850). Chimeric antibodies are produced by recombinant processes well known in the art, and have an animal variable region and a human constant region. Humanised antibodies have a greater degree of human peptide sequences than do chimeric antibodies. In a humanised antibody, only the complementarity determining regions (CDRs) which are responsible for antigen binding and specificity are animal derived and have an amino acid sequence corresponding to the animal antibody, and substantially all of the remaining portions of the molecule (except, in some cases, small portions of the framework regions within the variable region) are human derived and correspond in amino acid sequence to a human antibody. See L. Riechmann et al., Nature, 1988; 332: 323-327; G. Winter, United States Patent No. 5,225,539; C.Queen et al., U.S. 5,530,101. Deimmunised antibodies are antibodies in which the T and B cell epitopes have been eliminated, as described in WO9852976. They have reduced immunogenicity when applied in vivo. Human antibodies can be made by several different ways, including by use of human immunoglobulin expression libraries (Stratagene Corp., La Jolla, California) to produce fragments of human antibodies (VH, VL, Fv, Fd, Fab, or (Fab')2, and using these fragments to construct whole human antibodies using techniques similar to those for producing chimeric antibodies. Human antibodies can also be produced in transgenic mice with a human immunoglobulin genome. Such mice are available from Abgenix, Inc., Fremont, California, and Medarex, Inc., Annandale, New Jersey. One can also create single peptide chain binding molecules in which the heavy and light chain Fv regions are connected. Single chain antibodies ("ScFv") and the method of their construction are described in U.S. Patent No. 4,946,778. Alternatively, Fab can be constructed and expressed by similar means (M.J. Evans et al., J. Immunol. Meth., 1995; 184: 123-138). Another class of antibodies that may be used in the context of the present invention are heavy chain antibodies and derivatives thereof. Such single-chain heavy chain antibodies naturally occur in e.g. Camelidae and their isolated variable domains are generally referred to as "VHH domains" or "nanobodies". Methods for obtaining heavy chain antibodies and the variable domains are inter alia provided in the following references: WO 94/04678, WO 95/04079, WO 96/34103, WO 94/25591, WO 99/37681, WO 00/40968, WO 00/43507, WO 00/65057, WO 01/40310, WO 01/44301, EP 1134231, WO 02/48193, WO 97/49805, WO 01/21817, WO 03/035694, WO 03/054016, WO 03/055527, WO 03/050531, WO 01/90190, WO 03/025020, WO 04/041867, WO 04/041862, WO04/041865, WO 04/041863, WO 04/062551. All of the wholly and partially human antibodies are less immunogenic than wholly murine MAbs (or MAbs from other non-human animals), and the fragments and single chain antibodies are also less immunogenic. All these types of antibodies are therefore less likely to evoke an immune or allergic response. Consequently, they are better suited for in vivo administration in humans than wholly animal antibodies, especially when repeated or long-term administration is necessary. In addition, the smaller size of the antibody fragment may help improve tissue bioavailability, which may be critical for better dose accumulation in acute disease indications, such as tumor treatment or some viral infections.

### Pharmaceutical compositions

The invention further relates to a pharmaceutical preparation comprising as active ingredient a conjugate as herein defined above. The composition preferably at least comprises a pharmaceutically acceptable carrier (other than the carrier in the conjugate) in addition to the active ingredient (the conjugate). In some methods, the conjugate comprises a polypeptide or antibody of the invention as purified from mammalian, insect or microbial cell cultures, from milk of transgenic mammals or other source is administered in purified form together with a pharmaceutical carrier as a pharmaceutical composition. Methods of producing pharmaceutical compositions comprising polypeptides are described in US Patents No.'s 5,789,543 and 6,207,718. The preferred form depends on the intended mode of administration and therapeutic application. The pharmaceutical carrier can be any compatible, non-toxic substance suitable to deliver the polypeptides, antibodies or gene therapy vectors to the patient. Sterile water, alcohol, fats, waxes, and inert solids may be used as the carrier. Pharmaceutically acceptable adjuvants, buffering agents, dispersing agents, and the like, may also be incorporated into the pharmaceutical compositions. The concentration of the conjugate of the invention in the pharmaceutical composition can vary widely, i.e., from less than about 0.1% by weight, usually being at least about 1% by weight to as much as 20% by weight or more. For oral administration, the active ingredient can be administered in solid dosage forms, such as capsules, tablets, and powders, or in liquid dosage forms, such as elixirs, syrups, and suspensions. Active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable colour, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance. The conjugates of the invention are preferably administered parentally. Preparation with the conjugates for parental administration must be sterile. Sterilisation is readily accomplished by filtration through sterile filtration membranes, prior to or following lyophilisation and reconstitution. The parental route for administration of the conjugates is in accord with known methods, e.g. injection or infusion by intravenous, intraperitoneal, intramuscular, intraarterial, intralesional, intracranial, intrathecal, transdermal, nasal, buccal, rectal, or vaginal routes. The conjugate is administered continuously by infusion or by bolus injection. A typical composition for intravenous infusion could be made up to contain 10 to 500 ml of sterile 0.9% NaCl or 5% glucose optionally supplemented with a 20% albumin solution and the required dose of the conjugate. A typical pharmaceutical composition for intramuscular injection would be made up to contain, for example, 1 - 10 ml of sterile buffered water and the required dose of the conjugate of the invention. Methods for preparing parenterally administrable compositions are well known in the art and described in more detail in various sources, including, for example, Remington's Pharmaceutical Science (15th ed., Mack Publishing, Easton, PA, 1980).

For therapeutic applications, the pharmaceutical compositions are administered to a patient suffering from a viral infection or associated condition in an amount sufficient to reduce the severity of symptoms and/or prevent or arrest further development of symptoms. An amount adequate to accomplish this is defined as a "therapeutically-" or "prophylactically-effective dose". Such effective dosages will depend on the severity of the condition and on the general state of the patient's health.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there be one and only one of the elements. The indefinite article "a" or "an" thus usually means "at least one".

The following examples are offered for illustrative purposes only, and are not intended to limit the scope of the present invention in any way.

### Description of the figures

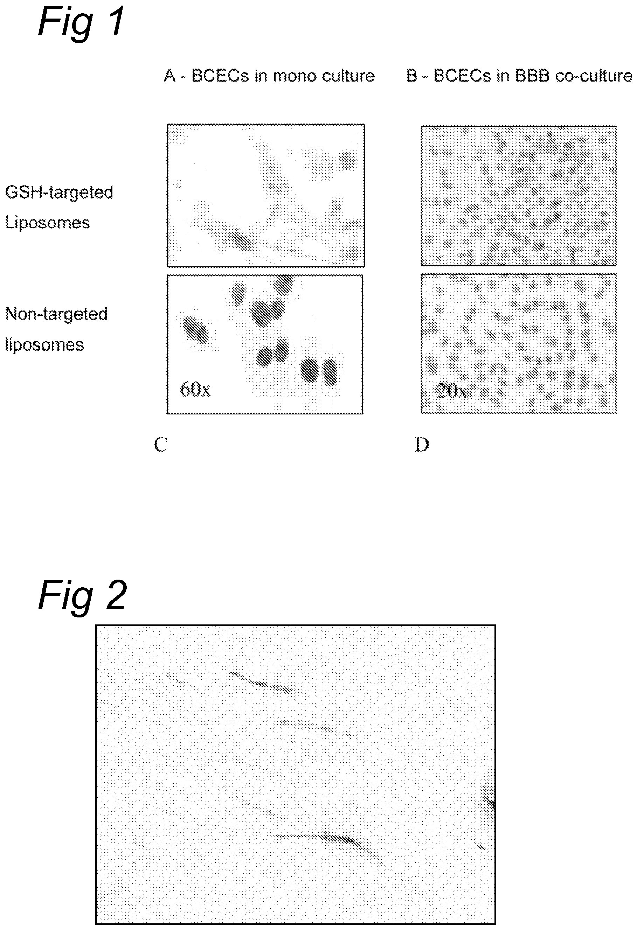

Figure 1 shows representative pictures of the uptake of glutathione-PEG-liposomes (labelled with Rho-PE) in BCEC. Shown is the uptake of GSH-targeted liposomes by Bovine capillary endothelial cells (BCECs) in BCECs monoculture (A) and in the BBB co-culture model (B). The micrographs show uptake of GSH-targeted liposomes (red) by BCECs cultures (nuclei counterstained in blue) after incubation times of ½ hr (A) and 2 hr (B). Incubation of BCECs monoculture (C) and in the BBB co-culture model (D) with non-targeted liposomes distinctly shows absence of red signal in or around the cells.
Figure 2 shows a picture of the specific targeting to the hamster brain of glutathione-PEG-liposomes (at the 50 mg/kg/day dosing regime), 3 days after the last intravenous daily bolus injection for 12 consecutive days. The micrograph above shows fluoresence signal of GSH-targeted liposomes mainly perivascular.

### Examples

### Reference Example 1

### Conjugation of agents to receptor-specific ligands

As an example of conjugation of agents to GSH, the preferred method of conjugation of GSH to protein drugs is disclosed. Similar conjugation chemistry is applied to the other herein disclosed agents, and the other herein disclosed GSH derivatives. In order to visualize the GSH-transporter specific cellular uptake, as well as the in vivo pharmacokinetics and biodistribution of GSH conjugated to a hydrophilic agent, GSH is labelled with the hydrophilic fluorescent dye fluorescein isothiocyanate (FITC) or Cy5.5. For this, GSH is dissolved in PBS and NaHCO3 pH 9.0. FITC or Cy5.5 is added and the solution is stirred, in the dark, for 1 hr at room temperature. The excess of FITC or Cy5.5 is removed by column centrifugation (Zeba™, Pierce, Rockford, USA) after which the solution is stored in the dark at 4°C.

### Example 2

### Conjugation of GSH-transporter specific ligands to nanocontainers containing encapsulated agents, and pharmacokinetics and pharmacodynamics thereof.

As an example of agent-containing nanocontainers coated with GSH-transporter specific ligands, the preferred method of conjugation of GSH to drug-loaded PEGylated liposomes is disclosed. Liposomes consist of phospholipids and cholesterol in several molar ratios (e.g., 2.0:1.5). In order to modify the transition/processing temperature and particle stability in plasma, phospholipids like 1,2-dipalmitoyl-sn-glycero-3-phosphocholine (DPPC), dimyristoylphosphatidylcholine (DMPC), hydrogenated soy phosphatidylcholine (HSPC), soy phosphatidylcholine (SPC), distearoyl phosphatidylcholine (DSPC), or egg yolk phosphatidylcholine (EYPC) are used in different ratios with cholesterol (Chol), where less Chol in the mixture will result in less stable liposomes in plasma. Components are dissolved in ethanol or isopropanol. Micelles containing DSPE-PEG-GSH (between 0.2 and 10 mol %), which is synthesized before preparation of the liposomes using DSPE-PEG-MAL and fresh solutions of reduced glutathione (rendering a MAL-reactive thiol group in the cysteine moiety of the tri-peptide) and DSPE-mPEG (Mw 2000) is added to the solution at different mol-percentages (up to 5-10 mol % in total). Alternatively, DSPE-PEG-GSH is conjugated using DSPE-PEG-NHS with activity towards the amine groups in GSH, or GSH is synthesized directly to DSPE-PEG at the N or C-terminal residue. When necessary for changing the electrical charge of the liposomes, dicetyl phosphate (DP) or DOTAP is added to the mixture. Additionally, nonionic surfactant polysorbate 80 (Tween 80)) may be added to the mixture. Also, other non-ionic surfactants may be used, like Tween 20, Tween 40, Brij76, Brij78 or those described in U.S. Patent 6,288,040 (i.e., carbodiimide, n-ethoxycarbonyl-2-ethoxy-1,2-dihycroquinoline, glutardioldehyde, bromozyane, meta-periodate (Na-salt or K-salt), tosyl chloride and chloroformic acid ester). The (lipid) mixture is injected in an aqueous solution containing the hydrophilic agent, with or without the presence of excipients or solubilizors like cyclodextrins. Lipophillic agents are added to the lipid mixture, or encapsulated (optionally as a post manufacturing process in the hospital pharmacy) using the active loading procedure with liposomes pre-filled with e.g., ammonium sulfate or calcium acetate. After vortexing, the vesicles are either extruded through membranes or homogenized in an emulsifier. Alternatively, DSPE-PEG-GSH is added after preparation of the liposomes by incubation at 25°C up to 60°C for 2 up to 24 hours (depending on the transition temperature of the lipid mixture and the temperature sensitivity of the agent). Liposomes are characterized by measuring particle size (50-200 nm on a Malvern Zetasizer), zeta potential, phospholipid content (using the Phopholipids B kit or HPLC/UPLC systems) and peptide content (0.2-10 mol% GSH based on HPLC/UPLC or an OPA assay of Pierce), and drug loading. This liposomal entrapment strategy is applied to the herein disclosed agents, and similar conjugation or synthesis chemistry the other herein disclosed GSH derivatives as ligands for targeting. In addition, similar liposomal entrapment is applied to the nucleic acid-based drugs, with additional enrichment of nucleic acid entrapment by addition of a cationic derivative of cholesterol (DC-Chol) to the liposomes, as detailed in Gao and Huang, 1991, Biochem Biophys Res Commun. 179(1):280-5, or by using amphoteric liposomes, as detailed in WO2002/066012. In order to visualize the receptor-specific cellular uptake, as well as the in vivo pharmacokinetics and biodistribution of GSH conjugated to the liposome filled with an agent, 1,2-dioleoyl-sn-glycero-3-phosphoethanolamine-N-lissamine Rhodamine B sulfonyl (Rho-PE) is added to the lipid mixture during the preparation of the liposomes. Alternatively, liposomes are labelled with radioactive tracer molecules, or the encapsulated agent is a fluorescent molecule (like Cy5.5) or a compound with a fluorescent probe (like Cy5.5 or FITC) conjugated to the agent.

For example, ribavirin, dissolved in PBS at 100 mg/ml at 50°C, was encapsulated in liposomes consisting of DPPC (55%), cholesterol (41%), rhodamine-PE (0.04%) and mPEG-DSPE (4.4%), dissolved in 2-propanol, using an emulsifier (Emulsiflex-C3). To test the influence of the amount of GSH molecules on the outer layer of the liposomes, different percentages of GSH-PEG-DSPE (0 - 2%) were prepared by a post-insertion of GSH-PEG-DSPE micelles into preformed liposomes in lieu of mPEG-DSPE, providing 0 or 0.1, 0.2, 0.5 or 2% GSH-PEG liposomes with a total PEG content of 5%. In this way, ribavirin GSH-PEG liposomes were 90 nm and contained 10 mg/ml ribavirin (encapsulation efficiency of 8-12%). Similar ribavirin GSH-PEG liposomes were prepared with EYPC instead of DPPC. The full (liposome-encapsulated) peak plasma levels of ribavirin in the 0% PEG liposomes (DPPC-based) after a single intravenous injection of 50 mg/kg in rats were found to be stable for several hours (half-life was estimated to be about 19 hours) at around 3 millimolar and free fraction in plasma was then around 4 micromolar. For the 2% GSH-PEG liposomes (DPPC-based), full plasma levels were also around 3 millimolar (with the same estimated half-life), where free fraction then just above 2 micromolar. In contrast, free ribavirin levels in the brain interstitual fluid (as determined by brain microdialysis) were just over 120 nanomolar for 0% PEG liposomes and around 600 nanomolar for 2% GSH-PEG liposomes. In addition, the enhanced brain delivery effect for the free ribavirin was found to be reduced by lowering the % of GSH on the liposomes: 450 nanomolar for 0.5%, 250 nanomolar for 0.2% and about 120 nanomolar for 0.1% GSH, which was again similar to 0%. These results indicate that the addition of GSH to the PEGylated liposomes enhances the delivery of free drug across the blood-brain barrier in a GSH-dependent manner, with a factor of about 5 times. Of relevance is that the area under the curve of the full plasma levels of ribavirin for the different formulations (0-2% GSH) was not significantly different. In contrast, the full (liposome-encapsulated) plasma level of ribavirin in EYPC-based liposomes (again after a single intravenous injection of 50 mg/kg in rats) was found to peak at only 1 millimolar and to rapidly decline (with a half-life of about 3 hours), with no observed difference between the 0 and 2% GSH on the outer surface of the liposomes. At the same time, the free fraction of ribavirin was found to be around 45 micromolar (peak value) and declined at the same rate as the full plasma level, again with no difference between the 0 and 2% GSH on the outer surface of the liposomes. These results indicate that DPPC-based liposomes have a long circulation time (and slow release of ribavirin) and the EYPC-based liposomes have a short circulation time (and a fast release of ribavirin).

As another example, PEGylated doxorubicin liposomes (based on Cealyx/Doxil) were modified to contain GSH on tips of the PEG. For this, GSH-PEG-DSPE micelles (5%) were dissolved in a 2 mg/mL ammonium sulfate solution at 60°C. To this solution, HSPC (55%) and cholesterol (40%) dissolved in ethanol (at 60°C) was added and liposomes were prepared by extrusion through filters until particles of about 100 nm were obtained. Subsequently, free ammonium sulfate was removed by dialysis and a 2 mg/mL doxorubicin solution was added (at 60°C), to allow for the doxorubicin to exchange with ammonium and precipitate within the liposome core ("active-loading"). The full (liposome-encapsulated) peak plasma levels of doxorubicin in the non-modified PEGylated liposomes (Caelyx/Doxil) after a single intravenous injection of 6 mg/kg in rats were found to be stable for several hours (half-life was estimated to be about 24 hours), where GSH-modified PEGylated liposomes displayed a faster clearance resulting in an estimated half-life of 19 hours. The results also show that the AUC obtained for non-modified PEGylated liposomes is about 50% greater than for the GSH-modified PEGylated liposomes, and Cmax values is about 20% higher. Sunsequently, these liposomes were evaluated for efficacy in athymic mice in which, using a stereotactic frame, a suspension of human glioblastoma cells (U87) was injected slowly into the brain. Mice were divided into three treatment groups: control (saline), doxorubicin in non-modified PEGylated liposomes (Caelyx/Doxil), and doxorubicin GSH-modified PEGylated liposomes. Animals received 5 mg/kg i.v. twice weekly. Treatment started 14 days after implantation of the U87 cells. No adverse effects (AEs) of the treatment were observed, the animals did not show any injection-related adverse events, nor did they show any neurological symptoms. The treatment with doxorubicin in GSH-modified PEGylated liposomes showed a strong significant reduction in brain tumour growth compared to saline and non-modified PEGylated liposomes in time (2-way ANOVA, p<0.001), and in a highly significant survival benefit when compared to the control groups (increase of 42% as compared to saline and 19% compared to non-modified PEGylated liposomes). In contrast, the same treatment groups (10 mg/kg i.v. every 4 days) were tested in nude mice with human metastatic breast cancer cells (MDA-MB-231) implanted under the skin, and no difference in treatment efficacy were observed between the two liposomal formulations; both were equally effective in reducing tumor burden when compared to vehicle treatment.

As yet another example, the same liposomal production process as described above for the doxorubicin GSH-modified PEGylated liposomes was applied to encapsulate the hemisuccinaat salts of methylprednisolon and deoxycorticosterone. For this, the ammonium sulfate solution was replaced by a calcium acetate solution, leading to high drug encapsulation efficiencies (>70%) and stable formulations. These formulations are tested for enhanced efficacy in CNS conditions (for instance in animal models for multiple sclerosis, or (stress-related) depression), and reduction of peripheral side effects (mainly cardiovascular) that are usually associated with these steroids.

As an example for peptide agents, the same liposomal production process as described above for the doxorubicin GSH-modified PEGylated liposomes was applied to encapsulate the beta-sheet breaker peptide LPFFP or Ac-LPFFP-NH2. For this, the ammonium sulfate solution was replaced by a 50-100 mg/mL solution of the peptide, leading to peptide encapsulation in the water core of the liposome with efficiencies of about 15% and a stable formulation. These formulations are tested for enhanced efficacy in CNS conditions in which amyloid-beta is involved (for instance in transgenic animal models for Alzheimer's Disease with enhanced plaque formation).

As an example for more sensitive protein agents, like antibodies, enzymes and growth factors, a lower processing temperature is preferred. For this, either between 95-55% EYPC, DMPC or DPPC, and between 0-40% cholesterol and 1% mPEG-DSPE was dissolved in ethanol at 37°C was added to protein solution of e.g., trastuzumab, gammaquin, cerezyme, elaprase, GDNF or albumin, stabilized with Tween 80 (<0.01%), and liposomes were prepared by extrusion through filters until particles of about 100 nm were obtained. Subsequently, 4-8% GSH-PEG-DSPE micelles were added at 37°C for 30 minutes up to 2 hours to the preformed liposomes, either during the production process or just prior to injection to a subject. Alternatively, 5% GSH-PEG-DSPE freeze-dried product was added to the lipid solution in lieu of the 1% mPEG-DSPE. When wanted, free protein was removed by dialysis/diafiltration or recaptured by protein specific columns (e.g., ProtG). The half-life of DMPC-based liposomes with 40% cholesterol was in the same range as the aforementioned half-life of the DPPC-based liposomes with 40% cholesterol. Reduction of the % of cholesterol to 10% shortened the plasma half-life (and thus IgG release) to about 2 hours, where omission of cholesterol from the formulation reduced half-life even further to about 30 minutes.

### Reference Example 3

### Conjugation of GSH to carrier for nucleid acid-based drugs

As an example of a non-viral delivery system for nucleic acid-based drugs by means of a targeted uptake mechanism, the preferred method of conjugation of PEGylated GSH to polyethylenimine (PEI), jetPEI, and the like or fragments thereof, is disclosed. PEGylated complexes are prepared as follows. PEI is dissolved in PBS. Poly(ethyleneglycol)-α-maleimide-ω-NHS (NHS-PEG-VS) is added to this solution and incubated at room temperature while mixing. The excess of NHS-PEG-VS is removed by ultrafiltration. PEI-PEG-VS is used directly for conjugation to the thiol group of reduced GSH.

### Reference Example 4

### Conjugation of GSH to proteins

As an example of a direct conjugation method, the preferred method of conjugation of GSH to enzymes, growth factors, monoclonal antibodies, or fragments thereof, is disclosed. GSH-cojugated proteins are prepared as follows. The amine groups of preferably lysine groups are modified with (sulfo)-SMCC rendering thiol-reactive maleimide groups on the protein. This reactive protein is subsequently used directly for conjugation to the thiol group of reduced GSH.

### Reference Example 5

### GSH-specific cell uptake and/or transcellular transport of targeted agents

GSH-specific cell uptake of the GSH conjugates is visualized by analysis of the specific uptake of the GSH conjugate, and compared to the level of uptake of control conjugates. Cells with a known (absence of) expression of GSH transporters are used from several species and origins, including porcine kidney epithelial cells (LLC-PK1), bovine brain capillary endothelial cells (BCEC), and canine MDCK cells. Cellular uptake experiments: 200 nmoles aliquots of targeted and non-targeted liposomes were added to an invitro model of blood brain barrier (co-cultures of rat astrocytes and bovine capillary endothelial cells) as well as to single cultures of BCECs. Incubation times ranged from ½ to 3hr at 37°C. At the end of the treatment cells on coverslips were fixed with 4% PFA and washed before being mounted on glass slides with Vectashield mounting medium containing DAPI (nuclear counterstain). The fate of the liposomes in the cell cultures was assessed by fluorescence microscopy using a NIKON TE2000-E inverted microscope, triple band filter for Rhodamine, GFP and DAPI at 20x or 60x magnification. Figure 1 shows the uptake of GSH-targeted liposomes by Bovine capillary endothelial cells (BCECs) in BCECs monoculture (plate A) and in the BBB co-culture model (plate B). The micrographs show uptake of GSH-targeted liposomes (Rho-PE in red) by BCECs cultures (nuclei counterstained in blue) after incubation times of ½ hr (A) and 2 hr (B). Incubation with non-targeted liposomes distinctly shows absence of red signal in or around the cells.

### Reference Example 6

### Pharmacokinetics and biodistribution of GSH-targeted agents

The pharmacokinetics and biodistribution of the GSH-targeted liposomes was visualized by analysis of the Rho-PE label in the liposomes after 12 daily intravenous bolus injections in hamsters, and compared to the un-targeted liposomes. The GSH-targeted liposomes showed a higher and specific accumulation in the perfused hamster brain, and less in a selection of other tissues analyzed (including heart, lung, liver, spleen and kidney), when compared to the control liposomes which were not (or hardly) detectable in brain, but to a relatively higher extend in lung, kidney and liver tissues 3 days after the last injection. Figure 2 shows a representative picture of a hamster brain slide from the higest dose group.

## Claims

1. A conjugate comprising:
a) a ligand for a glutathione transporter; wherein the ligand is selected from the group consisting of: glutathione, S-(p-bromobenzyl)glutathione, gamma-(L-gamma-azaglutamyl)-S-(p-bromobenzyl)-L-cysteinylglycin, S-butylglutathione, S-decylglutathione, glutathione reduced ethyl ester, glutathionesulfonic acid, S-hexylglutathione, S-lactoylglutathione, S-methylglutathione, S-(4-nitrobenzyl)glutathione, S-octylglutathione, S-propylglutathione, n-butanoyl gamma-glutamylcysteinylglycine, ethanoyl gamma-glutamylcysteinylglycine, hexanoyl gamma-glutamylcysteinylglycine, octanoyl gamma-glutamylcysteinylglycine, dodecanoyl gamma-glutamylcysteinylglycine, GSH monoisopropyl ester (N-(N-L-glutamyl-L-cysteinyl)glycine 1-isopropyl ester sulfate monohydrate) and glutathione derivatives of the formula I: 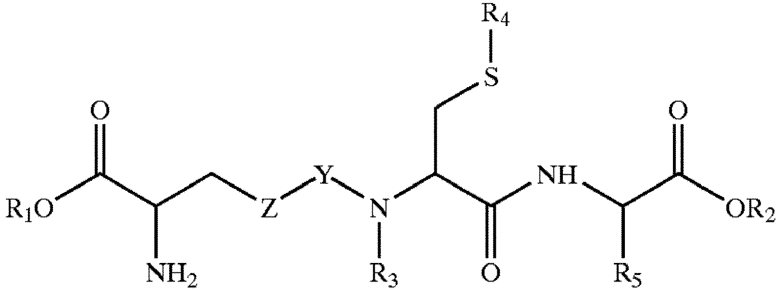
wherein Z=CH₂ and Y=CH₂, or Z=O and Y = C=O;
R₁ and R₂ are independently selected from the group consisting of H, linear or branched alkyl (1-25C), aralkyl (6-26C), cycloalkyl (6-25C), heterocycles (6-20C), ethers or polyethers (3-25C), and where R₁-R₂ together have 2-20C atoms and form a macrocycle with the remainder of formula I;
R₃ is selected from the group consisting of H and CH₃;
R4 is selected form the group consisting of 6-8C alkyl, benzyl, naphthyl and a therapeutically active compound; and,
R5 is selected from the group consisting of H, phenyl, CH₃- and CH₂-phenyl; or,
a pharmaceutically acceptable salt thereof,
and,
b) a liposome comprising an anthracycline,
wherein the ligand in a) is conjugated to the liposome in b).

2. A conjugate according to claim 1, wherein the anthracycline is selected from the group consisting of aclarubicin, daunorubicin, doxorubicin, epirubicin, idarubicin, amrubicin, pirarubicin, valrubicin, zorubicin.

3. A conjugate according to claim 1 or 2, wherein the ligand is a ligand that specifically binds to or is endocytosed or transcytosed into or through a BCEC or MDCK target cell at a rate that is at least 10% enhanced as compared control conditions selected from a) cells lacking expression of GSH transporters; b) cells pretreated with excess of free GSH; and c) a reference compound lacking a GSH moiety; when measured at 18 hours or less after addition of the ligand to the target cell.

4. A conjugate according to any one of the preceding claims, wherein in the derivative of formula I R₃ is H, R₄ is benzyl, and R₅ is phenyl.

5. A conjugate according to any one of the preceding claims for use in the treatment or prevention of a CNS disorder.

6. Use of a conjugate according to any one of the preceding claims for the manufacture of a medicament for the treatment or prevention of a CNS disorder.

## Patentansprüche

1. Konjugat, umfassend:
a) einen Liganden für einen Glutathiontransporter, wobei der Ligand ausgewählt ist aus der Gruppe bestehend aus Glutathion, S-(p-Brombenzyl)-Glutathion, gamma-(L-gamma-azaglutamyl)-S-(p-brombenzyl)-L-cysteinylglycin, S-Butylglutathion, S-Decylglutathion, Glutathion-reduziertem Ethylester, Glutathionsulfonsäure, S-Hexylglutathion, S-Lactoylglutathion, S-Methylglutathion, S-(4-Nitrobenzyl)-glutathion, S-Octylglutathion, S-Propylglutathion, n-Butanoyl-gamma-glutamylcysteinylglycin, Ethanoyl-gamma-glutamylcysteinylglycin, Hexanoyl-gamma-glutamylcysteinylglycin, Octanoyl-gamma-glutamylcysteinylglycin, Dodecanoyl-gamma-glutamylcysteinylglycin, GSH-Monoisopropylester (N-(N-L-Glutamyl-L-cysteinyl)-glycin-1-isopropylestersulfatmonohydrat) und Glutathionderivate der Formel I: 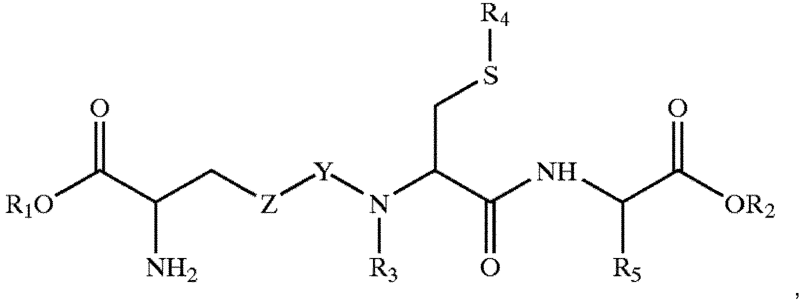
wobei Z=CH₂ und Y=CH₂ oder Z=O und Y = C=O,
R₁ und R₂ unabhängig voneinander ausgewählt sind aus der Gruppe bestehend aus H, linearem oder verzweigtem Alkyl (1-25C), Aralkyl (6-26C), Cycloalkyl (6-25C), Heterozyklen (6-20C), Ethern oder Polyethern (3-25C) und wobei R₁-R₂ zusammen 2-20 C-Atome haben und mit dem Rest der Formel I einen Makrozyklus bilden,
R₃ ausgewählt ist aus der Gruppe bestehend aus H und CH₃,
R4 ausgewählt ist aus der Gruppe bestehend aus 6-8C-Alkyl, Benzyl, Naphthyl und einer therapeutisch aktiven Verbindung und
R 5 ausgewählt ist aus der Gruppe bestehend aus H, Phenyl, CH₃- und CH₂-Phenyl,
oder ein pharmazeutisch verträgliches Salz davon
und
b) ein Liposom, das ein Anthracyclin umfasst,
wobei der Ligand in a) an das Liposom in b) konjugiert ist.

2. Konjugat gemäß Anspruch 1, wobei das Anthracyclin ausgewählt ist aus der Gruppe bestehend aus Aclarubicin, Daunorubicin, Doxorubicin, Epirubicin, Idarubicin, Amrubicin, Pirarubicin, Valrubicin, Zorubicin.

3. Konjugat gemäß Anspruch 1 oder 2, wobei der Ligand ein Ligand ist, der mit einer Rate, die im Vergleich zu Kontrollbedingungen, ausgewählt aus a) Zellen, denen es an Expression von GSH-Transportern mangelt, b) mit Überschuss an freiem GSH vorbehandelten Zellen und c) einer Referenzverbindung, der eine GSH-Einheit fehlt, um mindestens 10 % gesteigert ist, gemessen 18 Stunden oder weniger nach Zugabe des Liganden zur Zielzelle, spezifisch an eine BCEC oder MDCK-Zielzelle bindet oder in oder durch diese endozytiert oder transzytiert wird.

4. Konjugat gemäß einem der vorangehenden Ansprüche, wobei in das Derivat der Formel I R₃ H ist, R₄ Benzyl ist und R₅ Phenyl ist.

5. Konjugat gemäß einem der vorangehenden Ansprüche zur Verwendung in der Behandlung oder Prävention einer ZNS-Störung.

6. Verwendung eines Konjugats gemäß einem der vorangehenden Ansprüche zur Herstellung eines Arzneimittels zur Behandlung oder Prävention einer ZNS-Störung.

## Revendications

1. Conjugué comprenant :
a) un ligand pour un transporteur de glutathion ; dans lequel le ligand est choisi parmi le groupe constitué de : glutathion, S-(p-bromobenzyl)glutathion, gamma-(L-gamma-azaglutamyl)-S-(p-bromobenzyl)-L-cystéinylglycine, S-butylglutathion, S-décylglutathion, éthylester réduit par glutathion, acide glutathionsulfonique, S-hexylglutathion, S-lactoylglutathion, S-méthylglutathion, S-(4-nitrobenzyl)glutathion, S-octylglutathion, S-propylglutathion, n-butanoyl gamma-glutamylcystéinylglycine, ethanoyl gamma-glutamylcystéinylglycine, hexanoyl gamma-glutamylcystéinylglycine, octanoyl gamma-glutamylcystéinylglycine, dodécanoyl gamma-glutamylcystéinylglycine, GSH monoisopropylester (N-(N-L-glutamyl-L-cystéinyl)glycine 1-isopropylester sulfate monohydrate) and des dérivés de glutathion ayant la formule I : 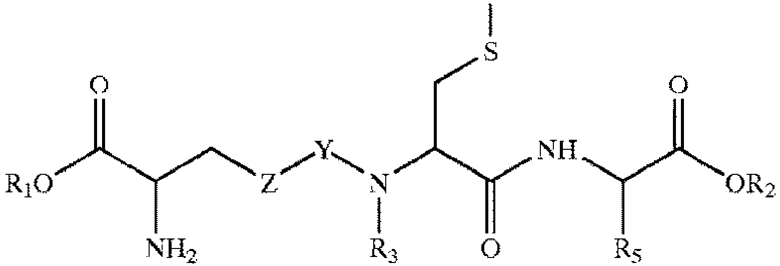
où Z = CH₂ et Y = CH₂, ou Z = O et Y = C=O ;
R₁ et R₂ sont choisis indépendamment parmi le groupe constitué de H, alkyle (1-25C), aralkyle (6-26C), cycloalkyle (6-25C), hétérocycles (6-20C), éthers ou polyéthers (3-25C) linéaires ou ramifiés, et où R₁-R₂ ont ensemble 2 à 20 atomes de carbone et forment un macrocycle avec le reste de la formule I ;
R₃ est choisi parmi le groupe constitué de H et CH₃ ;
R₄ est choisi parmi le groupe constitué d'alkyle en 6-8C, benzyle, naphtyle et d'un composé thérapeutiquement actif ; et,
R₅ est choisi parmi le groupe constitué de H, phényle, CH₃- et CH₂-phényle ;
ou d'un sel pharmaceutiquement acceptable de ceux-ci,
et,
b) un liposome comprenant une anthracycline,
dans lequel le ligand de a) est conjugué au liposome de b).

2. Conjugué selon la revendication 1, dans lequel l'anthracycline est choisie parmi le groupe constitué d'aclarubicine, daunorubicine, doxorubicine, épirubicine, idarubicine, amrubicine, pirarubicine, valrubicine, zorubicine.

3. Conjugué selon la revendication 1 ou 2, dans lequel le ligand est un ligand qui se lie spécifiquement à, ou effectue une endocytose ou transcytose dans ou à travers, une cellule cible BCEC ou MDCK à une vitesse qui est amplifiée d'au moins 10 % par comparaison à des conditions témoin sélectionnées parmi a) des cellules dépourvues d'expression de transporteurs de GSH ; b) des cellules prétraitées à l'aide d'un excès de GSH libre ; et c) un composé de référence dépourvu d'un fragment GSH ; lorsque mesurée 18 heures ou moins après ajout du ligand à la cellule cible.

4. Conjugué selon l'une quelconque des revendications précédentes, dans lequel dans le dérivé ayant la formule I, R₃ est H, R₄ est un benzyle, et R₅ est un phényle.

5. Conjugué selon l'une quelconque des revendications précédentes destiné à être utilisé dans le traitement ou la prévention d'un trouble du système nerveux central (CNS).

6. Utilisation d'un conjugué selon l'une quelconque des revendications précédentes, pour la fabrication d'un médicament destiné au traitement ou à la prévention d'un trouble du système nerveux central.
